(19) Europäisches Patentamt
European Patent Office
Office européen des brevets

(11) **EP 1 054 620 B1**

(12) **EUROPEAN PATENT SPECIFICATION**

(45) Date of publication and mention
of the grant of the patent:
**27.01.2010 Bulletin 2010/04**

(51) Int Cl.:
***A61B 5/00*** (2006.01)

(21) Application number: **99906976.8**

(86) International application number:
**PCT/US1999/003066**

(22) Date of filing: **12.02.1999**

(87) International publication number:
**WO 1999/040842 (19.08.1999 Gazette 1999/33)**

(54) **TRANSABDOMINAL EXAMINATION, MONITORING AND IMAGING OF TISSUE**

UNTERSUCHUNG, BEOBACHTUNG UND BILDDARSTELLUNG VON ABDOMINAL-GEWEBE

EXAMEN TRANSABDOMINAL, SURVEILLANCE ET IMAGERIE TISSULAIRES

(84) Designated Contracting States:
**DE FR GB**

(30) Priority: **13.02.1998 US 74642 P**
**26.08.1998 US 98172 P**
**26.08.1998 US 98018 P**

(43) Date of publication of application:
**29.11.2000 Bulletin 2000/48**

(73) Proprietor: **NON-INVASIVE TECHNOLOGY, INC.**
**Philadelphia, PA 19104 (US)**

(72) Inventor: **CHANCE, Britton**
**Marathon, FL 33050 (US)**

(74) Representative: **Wagner, Karl H.**
**Wagner & Geyer Partnerschaft**
**Patent- und Rechtsanwälte**
**Gewürzmühlstrasse 5**
**80538 München (DE)**

(56) References cited:
**WO-A-93/13395      WO-A-99/40840**
**US-A- 5 137 355      US-A- 5 551 424**
**US-A- 5 564 417      US-A- 5 845 639**
**US-A- 5 853 370**

**Description**

**The Field of the Invention**

The present invention relates to non-invasive, *in-vivo* examination, imaging and characterization of biological tissue using visible or infra-red radiation, and more particularly to transabdominal or transthoracic non-invasive examination, monitoring and imaging of internal tissue or an in utero fetus.

**Background**

Traditionally, X-rays or $\gamma$-rays has been used to examine and image biological tissue. This radiation propagates in the tissue on straight, ballistic tracks, i.e., scattering of the radiation is negligible. Thus, imaging is based on evaluation of the absorption levels of different tissue types. For example, in roentgenography the X-ray film contains darker and lighter spots. In more complicated systems, such as computerized tomography (CT), a cross-sectional picture of human organs is created by transmitting X-ray radiation through a section of the human body at different angles and by electronically detecting the variation in X-ray transmission. The detected intensity information is digitally stored in a computer which reconstructs the X-ray absorption of the tissue at a multiplicity of points located in one cross-sectional plane.

Near infra-red radiation (NIR) has been used to study non-invasively the oxygen metabolism in tissue (for example, the brain, finger, or ear lobe). Using visible, NIR and infra-red (IR) radiation for medical imaging could bring several advantages. In the NIR or IR range the contrast factor between a tumor and a tissue is much larger than in the X-ray range. In addition, the visible to IR radiation is preferred over the X-ray radiation since it is non-ionizing and thus, potentially causes fewer side effects. However, the visible or IR radiation is strongly scattered and absorbed in biological tissue, and the migration path cannot be approximated by a straight line, making inapplicable certain aspects of cross-sectional imaging techniques.

Computerized Tomography using NIR spectrometry has been used for *in vivo* imaging. This technique utilizes NIR radiation in an analogous way to the use of X-ray radiation in an X-ray CT. The X-ray source is replaced by several laser diodes emitting light in the NIR range. The NIR-CT uses a set of photodetectors that detect the light of the laser diodes transmitted through the imaged tissue. The detected data are manipulated by a computer similarly as the detected X-ray data would be in an X-ray CT. Different NIR-CT systems have recognized the scattering aspect of the non-ionizing radiation and have modified the X-ray CT algorithms accordingly.

The above-mentioned X-ray or $\gamma$-ray techniques have been used to detect a tissue tumor. Under the term "angiogenesis" I mean the generation of new blood vessels into a tissue or organ. Under normal physiological conditions humans or animals undergo angiogenesis only in very specific restricted situations. For example, angiogenesis is normally observed in wound healing, fetal and embryonal development and formation of the corpus luteum, endometrium and placenta.

Both controlled and uncontrolled angiogenesis are thought to proceed in a similar manner. Persistent, unregulated angiogenesis occurs in a multiplicity of disease states, tumor metastasis and abnormal growth by endothelial cells and supports the pathological damage seen in these conditions. The diverse pathological disease states in which unregulated angiogenesis is present have been grouped together as angiogenic dependent or angiogenic associated diseases. The hypothesis that tumor growth is angiogenesis dependent was first proposed in 1971. (Folkman J., Tumor Angiogenesis: Therapeutic Implications., N. Engl. Jour. Med. 285: 1182-1186, 1971) In its simplest terms it states: "Once tumor 'take' has occurred, every increase in tumor cell population must be preceded by an increase in new capillaries converging on the tumor." Tumor 'take' is understood to indicate a prevascular phase of tumor growth in which a population of tumor cells occupying a few cubic millimeters volume and not exceeding a few million cells, can survive on existing host microvessels. Expansion of tumor volume beyond this phase requires the induction of new capillary blood vessels. This explanation was directly or indirectly observed and documented in numerous publications.

Ultrasound systems are widely used for in utero examination of a fetus. However, these systems are not very sensitive to tissue oxygenation. Perinatal brain injury, such as hypoxic - ischemic encephalopathy (HIE) and germinal-matrix intraventricular hemorrhage (GM-IVH), is still a significant source of neurological morbidity, cerebral palsy (CP), mental retardation, and seizures. Premature fetuses and infants are particularly at high risk of developing brain injury. GM-IVH, a common problem related to prematurity, is a potent risk factor for CP. The overall incidence of CP is approximately 1 to 2 per 1000 live births; however, the incidence dramatically increases with prematurity, i.e. 15 per 1000 live births for those weighing less than 2500g, and from 13 to 90 per 1000 survivors from 500-1500g.

None of the existing diagnostic methods for fetal surveillance provides very accurate information on fetal cerebral hemodynamics and oxygenation. Antepartum electronic fetal heart rate (FHR) monitoring, either alone (non stress test - NST) or as the part of the biophysical profile (BPP), has been the primary means of assessing fetal health in the United States for decades. NST can forecast severe fetal jeopardy [23], but false-positive NST rates in excess of 90% have been reported. On the other hand, only high (>8) BPP scores and low (zero) BPP scores were predictive of normal pH and academic babies respectively, while the BPP score of 6 was a poor predictor of abnormal outcome.

Intrapartum fetal heart rate (FHR) monitoring is a standard of care in the United States. FHR has turned to be a poor predictor of neurological outcome, failing substantially to fulfill the major purpose of using any particular technique: the avoidance of neurological sequelac. Frequently, FHR monitoring has led to unnecessary interference with the birth process, and even to harm through increased rate of cesarean sections. Recent trials using pulse oximetry in the human fetus during labor may provide some insight in fetal oxygenation during labor, but the relevance of scalp or face oxygenation to cerebral oxygenation and hemodynamics should be taken with great caution, especially as it has been shown that the circumstances exist where cerebral hypoxia may develop in the presence of appropriate peripheral arterial and venous oxygen saturation.

Optical spectroscopy could be used to monitor and image tissue blood oxygenation and volume by measuring absorption of oxyhemoglobin and deoxyhemoglobin in the near infrared (NIR) wavelength region. Below 700 nm, light is strongly absorbed by hemoglobin. Above 900 nm, it is strongly absorbed by water. By making differential measurements at either side of the isosbestic point of oxy-hemoglobin and deoxy-hemoglobin absorbance (near 800 nm), it is possible to quantify the blood oxygenation and volume levels. Typically, these measurements are made at 750 nm and 830 nm.

Optical spectroscopy has been used to monitor an intra-partum fetus. Delpy et al. have demonstrated the possibility of intrapartum optical monitoring in human fetuses by using a continuous wave (CW) optical instrument (Hamamatsu NIRO-500 Monitor) and soft rubber probes placed through the cervix of the laboring woman and up against the fetal head to carry small fiber optic cables transmitting and receiving NIR light (Peebles, D.M. et al., "Changes in human fetal cerebral hemoglobin concentration and oxygenation during labor measured by near-infrared spectroscopy", Am. J. Obstet. Gynecol, 1992; 166:1369-73). They have reported changes in fetal cerebral oxygenation (cerebral desaturation) following variable, late, and prolonged decelerations, (Aldrich, C.J. et al., "Late fetal heart rate decelerations and changes in cerebral oxygenation during the first stage of labour", Br. J. Obstet. Gynaecol. 1995a; 102:9-13); (Aldrich, C.J. et al., "Fetal heart rate changes and cerebral oxygenation measured by near infrared spectroscopy during the first stage of labour", E. J. Obstet. Gynecol. Reprod. Biol., 1996; 64:189-195) as well as with short contraction intervals (Peebles, D.M. et al., "Relation between frequency of uterine contractions and human fetal cerebral oxygen saturation studied during labour by near-infrared spectroscopy", Br. J. Obstet. Gynaecol., 1994; 101:44-48). A significant correlation between cerebral oxygen saturation measured by optical spectroscopy shortly before delivery and fetal umbilical blood gas and acid-base status at birth has been reported, (Aldrich, C.J. et al., "Fetal cerebral oxygenation measured by near-infrared spectroscopy shortly before birth and acid-base status at birth", Obstet. Gynecol., 1994a; 84:861-6) as well as a significant rise in fetal cerebral oxygenation after maternal oxygen administration during normal labor (Aldrich, C.J. et al., "The effect of maternal oxygen administration on human fetal cerebral oxygenation measured during labour by near infrared spectroscopy", Br. J. Obstet. Gynaecol, 1994b; 101:509-513). Changes in maternal posture during labor, in women with effective epidural analgesia, were reportedly associated with a significant decrease in fetal cerebral oxygenation (Aldrich, C.J. et al., "The effect of maternal posture on fetal cerebral oxygenation measured during labour by near infrared spectroscopy", Br. J. Obstet. Gynaecol., 1995b; 102:14-19). Paul Mannheimer (Mannheimer, P.D. et al., "Physio-optical considerations in the design of fetal pulse oximetry sensors", Euro. J. Obstet. & Gyn., 1997; S9-S 19) (Reference Notem: "Nellcor Puritan Bennett N-400 fetal oxygen saturation monitoring system: technical issues", Nellcor Puritan Bennett, Inc., Perinatal Note Number 1, Pleasanton, CA 94588) and Swedlow (Swedlow, D.B., Reference Notem: "Nellcor Puritan Bennett N-400 review of evidence for a fetal SpO2 critical threshold of 30%", Nellcor Puritan Bennett, Inc., Perinatal Note Number 2, Pleasanton, CA 94588) have recently published design considerations and recommended limiting arterial desaturation values (30%) for the fetal brain.

There is still a need for a non-invasive, relatively inexpensive technique that can detect, image and characterize a tumor. Furthermore, there is still a need for a non-invasive, relatively inexpensive technique that can examine and monitor an in utero fetus.

## Summary

PCT publication WO 93/13395 discloses a spectrophotometer for providing an image of a radiation scattering medium having one or more radiation attenuating constituents is disclosed herein. The spectrophotometer includes a light source for illuminating the scattering medium with electromagnetic radiation of at least one wavelength. The spectrophotometer may also a time-gated detector serves to detect, during a predefined detection interval, the electromagnetic radiation having traversed a distribution of path lengths during propagation through a region of the medium. A photon counting apparatus or the like measures the intensity of the detected portion of the electromagnetic radiation, wherein the measured intensity is a function of attenuation of the region within the medium. A display apparatus is operative to generate the image in accordance with the measured intensity.

US Patent 5,564,417 discloses a pathlength corrected spectrophotometer for tissue examination includes an oscillator for generating a carrier waveform of a selected frequency, an LED light source for generating light of a selected wavelength that is intensity modulated at the selected frequency introduced to a subject, and a photodiode detector for detecting light that has migrated in the tissue of the subject. The spectrophotometer also includes a phase detector for measuring a phase shift between the introduced and detected light, a magnitude detector for determination of light attenuation in the examined tissue, and a processor adapted to calculate the photon migration pathlength and determine a physiological property of the examined tissue based on the pathlength and on the attenuation data.

US Patent 5,551,424 discloses a fetal probe that is insertable through the mother's vagina and cervixes and connectable to a pulse oximeter for monitoring the EKG and oxygen saturation of hemoglobin of a fetus during labor and delivery. The probe incorporates at least one spiral electrode and optical fiber or fibers and/or solid state light sources for monitoring oxygen saturation. Light emitting diodes, or an optical fiber or fiber pair, transmit light of two different wavelengths through the fetal skin to perfused fetal tissue while either the EKG needle or another needle extends into fetal tissue and contains a light collecting optical fiber having a distal light collecting surface adjacent the relative needle distal end for collecting transmitted light that has passed through perfused fetal tissue that is interior of the fetal skin.

US Patent 5,807,263 discloses a method and system for optical examination of a subject positioned between input and detection ports. The optical system includes at lease one light source for introducing at one or multiple input ports, electromagnetic non-ionizing radiation of a known time-varying pattern of photon density of a wavelength selected to be scattered and absorbed while migrating in the subject. The optical system also includes radiation pattern control means for achieving a directional pattern of emitted resulting radiation that possesses substantial gradient of photon density, and at least one detector for detecting the radiation that has migrated in the subject at one or multiple detection ports. The system also includes processing means for processing the detected radiation and creating sets of data, and evaluation means for examining the subject using the data sets. The emitted directional radiation pattern utilizes its gradient of photon density to detect a hidden object while scanning across the examined subject. The wavelength of the radiation can be selected to be sensitive to endogenous or exogenous pigments, or to cause fluorescent emission from a fluorescent constituent of interest in the subject. The operation of the system is computer controlled.

US Patent 5,673,701 discloses optical methods and systems for examination of a subject positioned between input and detection ports. The spectroscopic system includes at least one least source that introduces at one or multiple input ports electromagnetic non-ionizing radiation into the subject of a visible or infrared wavelength selected to be scattered and absorbed while migrating in the tissue. The spectroscopic system also includes a detector optically connected to the detection port, wherein the detector is connected to a detector circuit. A controller selects radiation intensities to be introduced to the tissue, wherein the selected radiation intensities define a null plane in the tissue. The detection port is positioned relative to a selected detection location of the examined tissue corresponding to the null plane. The optical radiation of the first intensity is introduced into the subject, at the first input port, and the first radiation that has migrated in the examined tissue is detected. The detector circuit stores a first detector signal corresponding to the first detected radiation. The optical radiation of the second intensity is introduced into the subject, at the second input port and the second radiation that has migrated in the examined tissue is detected. The detector circuit stores a second detector signal corresponding to the second detected radiation. The first detector signal is subtracted from the second detector signal to obtain processed data.

US Patent 5,853,370 discloses an optical system for in vivo, non-invasive imaging of tissue change. The system includes an optical module, a spectrophotometer, and a processor. The optical module includes an array of input ports and detection ports located in a selected geometrical pattern to provide a multiplicity of arrayed source-detector pairs engaged directly with the subject. The spectrophotometer includes at least one light source constructed to introduce electromagnetic radiation of visible or infra-red wavelength into the examined tissue successively at the input ports, wherein the wavelength is sensitive to a constituent of the imaged tissue, and at least one detector constructed to detect, at the detection ports, radiation of the selected wavelength that has migrated in the tissue from respective input ports. The processor receives signals of the detected radiation from the detector, and is constructed and arranged to create a defined spatial image of the tissue by effectively producing from signals from the multiplicity of photon migration paths in the tissue, a succession of data sets representing, from a selected view, a succession of spatial images of the tissue being stimulated and the tissue not being stimulated, and an image data set related to differences between data of the successive data sets of the stimulated and non-stimulated tissue.

## Summary

The present invention relates to an apparatuses and method for non-invasive optical examination, imaging and monitoring of internal tissue using visible or infra-red light. The invention also relates to non-invasive optical examination, imaging and monitoring of an in utero fetus or fetal tissue, as defined in the independent claims.

According to one preferred aspect, the optical examination technique employs an optical system for *in vivo*, non-invasive examination of biological tissue of a subject. The optical system includes an optical module, a controller, and a processor. The optical module includes an array of optical input ports and detection ports located in a selected geometrical pattern to provide a multiplicity of photon migration paths inside an examined region of the biological tissue. Each optical input port is constructed to introduce visible or infrared light emitted from a light source. Each optical detection port is constructed to receive photons of light that have migrated in the examined tissue region from at least one of the input ports and provide the received light to a light detector. The controller is constructed and arranged to control operation of the light source and the light detector to detect light that has migrated over at least one of the photon migration paths. The processor is connected to receive signals from the detector and arranged to form at least two data sets, a first of the data sets representing blood volume in the examined tissue region and a second of the data sets representing blood oxygenation in the examined tissue region. The processor is arranged to correlate the first and second data sets to detect abnormal tissue in the examined tissue region.

Preferably, the second data set includes hemoglobin deoxygenation values. The processor may be arranged to form a third data set being collected by irradiating a reference tissue region.

According to another preferred aspect, the optical examination technique employs an optical system for *in vivo*, non-invasive examination of biological tissue of a subject. The optical system includes an optical module, a controller, and a processor. The optical module includes an array of optical input ports and detection ports located in a selected geometrical pattern to provide a multiplicity of photon migration paths inside an examined region of the biological tissue. Each optical input port is constructed to introduce visible or infrared light emitted from a light source. Each optical detection port is constructed to receive photons of light that have migrated in the tissue from at least one of the input ports and provide the received light to a light detector. The controller is constructed and arranged to control operation of the light source and the light detector to detect light that has migrated over at least one of the photon migration paths. The processor is connected to receive signals from the detector and arranged to form at least two data sets, a first of the data sets being collected by irradiating an examined tissue region of interest and a second of the data sets being collected by irradiating a reference tissue region having similar light scattering and absorptive properties as the examined tissue region. The processor is arranged to correlate the first and second data sets to detect abnormal tissue in the examined tissue region.

According to another preferred aspect, the optical examination technique employs an optical system for *in vivo*, non-invasive examination of biological tissue of a subject. The optical system includes an optical module, a controller, and a processor. The optical module includes an array of optical input ports and detection ports located in a selected geometrical pattern to provide a multiplicity of photon migration paths inside an examined region of the biological tissue or a model representing biological tissue. Each optical input port is constructed to introduce visible or infrared light emitted from a light source. Each the optical detection port is constructed to receive photons of light that have migrated in the tissue or the model from at least one of the input ports and provide the received light to a light detector. The controller is constructed and arranged to control operation of the light source and the light detector to detect light that has migrated over at least one of the photon migration paths. The processor is connected to receive signals from the detector and arranged to form at least two data sets of two tissue regions, a first of the data sets being collected by irradiating an examined tissue region and a second of the data sets being collected by irradiating a region of a tissue model having selected light scattering and absorptive properties. The processor is arranged to correlate the first and second data sets to detect abnormal tissue in the examined tissue region.

Preferred embodiments of these aspects include one or more of the following features.

The processor may be arranged to correlate the first and second data sets by determining congruence between data of the two data sets.

The processor may be programmed to order the first and second data sets as two-dimensional images and to determine the congruence using the two-dimensional images.

The processor may be programmed to order the first and second data sets as two-dimensional images and to determine the congruence using the following formula:

$$1 - \left( \frac{maximum\ overlap\ residual}{maximum\ selected\ tissue\ signal} \right) \times 100$$

The processor may be further arranged to determine a location of the abnormal tissue within the examined tissue region.

The processor may be adapted to produce from the data set an image data set by implementing an optical tomography algorithm. The optical tomography algorithm may use factors related to determined probability distribution of photons attributable to the scattering character of the tissue being imaged.

The controller may be arranged to activate the source and the detector to obtain a first selected distance between the input and detection ports, and the processor may be arranged to form the data set for the first distance. The processor may produce an image data set from the data set formed for the first distance. The controller may further be arranged to activate the source and the detector to obtain a second selected distance between the input and detection ports and is arranged to form another data set for the second distance.

The optical system may further include a display device constructed to receive the image data set from the processor and to display an image.

The optical system may further include a first oscillator and a phase detector. The first oscillator is constructed to generate a first carrier waveform at a first frequency on the order of $10^8$ Hz, the first frequency having a time characteristic compatible with the time delay of photon migration from the input port to the detection port. The light source is coupled to the first oscillator and constructed to generate the light modulated by the first carrier waveform. The phase detector is constructed to determine change in waveform of the detected light relative to the waveform of the introduced light and measure therefrom the phase shift of the detected light at the wavelength, wherein the phase-shifted light is indicative of scattering or absorptive properties of the examined tissue region. The processor is arranged to form the data set based on the measured phase shift. This optical system may further include a second oscillator constructed to generate a second waveform at a second frequency. The detector is then arranged to receive a reference waveform at a reference frequency offset by a frequency on the order of $10^3$ Hz from the first frequency and to produce a signal, at the offset frequency, corresponding to the detected radiation. The phase detector is adapted to compare, at the offset frequency, the detected radiation with the introduced radiation and to determine therefrom the phase shift.

The optical system may further include an oscillator, a phase splitter, and first and second double balanced mixers. The oscillator is constructed to generate a first carrier waveform of a selected frequency compatible with time delay of photon migration from the input port to the detection port The light source is connected to receive from the oscillator the carrier waveform and is constructed to generate optical radiation modulated at the frequency. The phase splitter is connected to receive the carrier waveform from the oscillator and produce first and second reference phase signals of predefined substantially different phases. The first and second double balanced mixers are connected to receive from the phase splitter the first and second reference phase signals, respectively, and are connected to receive from the detector the detector signal and to produce therefrom a in-phase output signal and a quadrature output signal, respectively. The processor being connected to the double balanced mixers and arranged to receive the in-phase output signal and the quadrature output signal and form therefrom the data set.

The processor may be arranged to calculate a phase shift ($\Theta_\lambda$) between the light introduced at the input port and the light detected at the detection port prior to forming the data set.

The processor may arranged to calculate an average migration pathlength of photons scattered in the examined tissue between the optical input port and the optical detection port prior to forming the data set.

The processor may further employ the pathlength in quantifying hemoglobin saturation (Y) of the examined tissue.

The processor may be arranged to calculate a signal amplitude ($A_\lambda$) determined as a square root of a sum of squares of the in-phase output signal and the quadrature output signal prior to forming the data set.

The optical system may further include a narrow band detector connected to receive from the optical detector the detector signal and to produce a DC output signal therefrom. The processor then further determines a modulation index ($M_\lambda$) as a ratio of values of the signal amplitude and the signal amplitude plus the DC output signal.

The optical system may further include at least one oscillator connected to at least one light source. The oscillator is constructed to generate a carrier waveform of a selected frequency. The light source generate slight of a visible or infrared wavelength being intensity modulated at the frequency to achieve a known light pattern. The controller is constructed to control the emitted light intensity or phase relationship of patterns simultaneously introduced from multiple input ports, wherein the introduced patterns form resulting radiation that possesses a substantial gradient of photon density in at least one direction. This resulting radiation is scattered and absorbed over the migration paths. The detector is constructed and arranged to detect over time the resulting radiation that has migrated in the tissue to the detection port. The processor is further arranged to process signals of the detected resulting radiation in relation to the introduced radiation to create the data sets indicative of influence of the examined tissue upon the substantial gradient of photon density of the resulting radiation.

The optical system may further include a phase detector constructed to detect the phase of the detected radiation and provide the phase to the processor.

The optical system may further include an amplitude detector constructed to detect the amplitude of the detected radiation and provide the amplitude to the processor.

The phase relationship of light patterns introduced from two input ports may be 180 degrees.

The optical system may be constructed as described in U.S. Patent 5,119,815 or 5, 386,827. This system includes a light source constructed to generate pulses of radiation of the wavelength, the pulses having a known pulse wave form of a duration on the order of a nanosecond or less. An optical detector is constructed to detect over time photons of modified pulses that have migrated in the tissue from the input ports. This system also includes an analyzer connected to the detector and adapted to determine a change in the pulse waveform shape of the detected pulses relative to the introduced pulses, at the employed wavelength. The processor then creates the data set based on the determined pulse waveform change. The processor may also be constructed and arranged to calculate the effective pathlength of photons of the wavelength migrating between the input and detection ports in conjunction with creating the data set. The processor may also be constructed and arranged to calculate the scattering coefficient at the wavelength in conjunction with creating the image data set The processor may also be constructed and arranged to calculate the absorption coefficient at the wavelength in conjunction with creating the data set.

The optical system may use the light source that produces relatively long light pulses and the processor that forms the data set by subtracting amplitude of two the pulses emitted from two input ports located symmetrically relative to one detection port.

The optical system may be constructed to introduce and detect photons at two wavelengths selected to be sensitive to a tissue constituent. The tissue constituent may be an endogenous pigment or an exogenous pigment. The endogenous pigment may be hemoglobin. The exogenous pigment may be a selected contrast agent.

According to another aspect, an optical apparatus for *in vivo,* non-invasive, transabdominal examination of fetal tissue includes an optical module, a controller, and a processor. The optical module includes an array of optical input ports and detection ports located in a selected geometrical pattern to provide a multiplicity of photon migration paths inside the uterus. Each optical input port is constructed to introduce visible or infrared light emitted from a light source. Each optical detection port is constructed to receive photons of light that have migrated from at least one of the input ports and provide the received light to a light detector. The controller constructed and arranged to control operation of the light source and the light detector to detect photons that have migrated over at least one of the photon migration paths inside fetal tissue. The processor connected to receive signals from the detector and arranged to characterize the fetal tissue region.

Preferred embodiments of this aspect include have one or more of the following features.

The controller and the processor may be arranged to evaluate the optical data and subsequently control operation of the light source and the light detector to collect additional optical data corresponding to photons that have partially migrated inside brain tissue of the fetus.

The optical module may be constructed for placement on the abdomen based on locating the head of the fetus by an ultrasound system so that the optical data correspond to photons that have partially migrated inside brain tissue of the fetus.

The processor may be arranged to determine hemoglobin oxygenation of the fetal tissue or a pulse rate of the fetus. The processor may be arranged to create an image the brain tissue. The processor may be arranged to create images blood volume in the brain tissue and blood oxygenation in the brain tissue.

According to another preferred aspect, an optical method for *in vivo*, non-invasive, transabdominal examination of fetal tissue is provided, as defined in claim 1.

According to another preferred aspect, the described optical techniques can be used to examine, monitor or image selected tissue of an in utero fetus. To collect the optical data, the techniques can employ different optical modules designed for targeting *in vivo*, non-invasively the fetus. The optical module includes an array of optical input ports and optical detection ports located over selected geometrical patterns that provide a multiplicity of photon migration paths. The photon migration paths partially include the selected fetal tissue such as the fetus's brain. The optical module may be moved around the exterior of the abdomen to locate the selected tissue of the fetus (e.g., the head) within the photon migration paths. The optical apparatus can provide single wavelength or multiple wavelength data of the fetal tissue, wherein an employed wavelength is sensitive to absorption or scattering by a tissue constituent (e.g., hemoglobin, or an introduced contrast agent). The optical apparatus may also generate blood volume, hemoglobin oxygenation or hemoglobin deoxygenation data, or data sensitive to any other tissue constituent. Based on the optical data, the apparatus may also measure the heart rate of the fetus, for example, by techniques used in pulse oximetry.

The optical imaging apparatus can further generate blood volume, hemoglobin oxygenation or hemoglobin deoxygenation images, or images of any other tissue constituent based on single or multiple wavelength optical data. The apparatus can use different images processing and enhancing algorithms known in the art. The apparatus can be used for a short term or prolonged transabdominal monitoring or for routine examination of the fetus. The apparatus may also be used for monitoring while in labor, wherein a clinician makes a decision about the state of the fetus based on the optical data.

The optical system can also include a stimulator constructed to stimulate a selected functional activity of the examined fetal tissue. The stimulator is constructed to deliver electrical signals, electromagnetic signals, vibroaccoustic signals, or sound such as loud rhythmic music to the fetus. Alternatively, the stimulator can deliver chemical substances to the fetus. For example, oxytocin may be administered intravenously to the pregnant female to induce uterine contractions. The pregnant female can ingest cold liquids or carbohydrates (fructose, glucose, complex carbohydrates), or can change her body position creating changes in uterine pressure to stimulate the fetus. The optical system can collect data before, during and after the simulation.

The described optical techniques can also be used in combination with ultrasound techniques, X-ray techniques (including CT), or magnetic resonance imaging (MRI or *f*MRI). These techniques may be used acquire data that are correlated with the optical data. To collect the optical data, the optical apparatus may employ different optical modules suitable for targeting the fetus at a different developmental stage. The optical modules include an array of light sources and light detectors located in a selected geometrical patterns to provide a multiplicity of source-detector paths of photon migration inside the examined fetal organ. For example, the ultrasound technique is used to locate the fetal heart and then the described optical technique can non-invasively characterize the blood volume and oxygenation.

The described optical imaging systems may generate single wavelength or multiple wavelength images of the examined tissue, wherein the used wavelength is sensitive to absorption or scattering by a tissue constituent (e.g., an endogenous or exogenous pigment, tissue cells) or is sensitive to structural changes in the tissue. The optical images may display tissue absorption, tissue scattering or both. The optical imaging systems may also generate blood volume and hemoglobin deoxygenation images of the examined organ, or may generate images of any other tissue constituent based on multiple wavelength optical data. A processor may employ different image processing and enhancing algorithms known in the art.

The optical imaging system may collect single wavelength or multiple wavelength data of a tissue model for calibration, or for detection of background data. In the calibration procedure, the optical module is placed on the model and the imaging system collects a limited number of optical data or collects optical data using the same sequences as used during the tissue examination. The system may either store the model data for a subsequent digital processing, or may adjust the source or detector gains to detect optical data according to a selected pattern. The imaging system may use different organ models having the same scattering coefficient or the same absorption coefficient as the normal tissue of the organ. The model may also include a representation of the abdominal wall (or a representation of other "obscuring" tissue structures, such as blood vessels, organs, or ribs) having the same scattering coefficient and the same absorption coefficient as the abdominal wall. The model tissue may have the scattering and absorption coefficient of abnormal or infected tissue approximating an examined organ. Furthermore, the models may have different sizes and shapes.

In general, an optical examination technique employs an optical system for *in vivo* non-invasive imaging of a region of biological tissue of a subject. The optical imaging system includes an optical module, a controller and a processor. The optical module includes an array of optical input ports and optical detection ports located in a selected geometrical pattern to provide a multiplicity of photon migration paths inside the biological tissue of interest. Each optical input port is constructed to introduce into the tissue volume visible or infrared light emitted from a light source. Each optical detection port is constructed to provide light from the tissue to a light detector. The controller is constructed and arranged to activate one or several light sources and light detectors so that the light detector detects light that has migrated over at least one of the photon migration paths. The processor receives signals corresponding to the detected light and creates at least one data set representing the examined tissue. The processor may also produce a spatial image of the examined tissue region.

To characterize the examined tissue, the imaging system can correlate several images of blood volume, hemoglobin oxygenation, hemoglobin deoxygenation, or images sensitive to an optical contrast agent. The imaging system can correlate images of the same tissue region taken at different times. The imaging system can correlate images of "symmetric" tissue, such as tissue of different region of the same organ (e.g., the liver, the lungs) or symmetric organs (e.g., the right and left kidney, the right and left brain hemisphere, the right and left leg, the right and left arm). The imaging system can correlate images of tissue with images of a tissue model. The correlation of the images identifies pathological tissue regions, such as tumors undergoing angiogenetic growth or hypermetabolism, wherein the tumor area exhibits an increased blood volume and decreased hemoglobin oxygenation. Furthermore, the correlation of the images can be used to monitor inhibition of angiogenesis during or after drug treatment.

To collect the optical data, the described optical examination techniques can use one or several optical modules having different design. The optical modules are constructed to target a selected tissue region of the examined organs by specific geometrical patterns of source-detector photon migration paths. Each source is displaced from one or several detectors by a spacing between about 1 cm and 25 cm (preferably 4 cm and 15 cm and more preferably 10 cm) to establish a "banana-shaped" or a "cigar-shaped" probability gradient of migrating photons in the tissue. Alternatively, each detector is displaced from one or several sources by a spacing between about 1 cm and 25 cm (preferably 4 cm and 15 cm, and more preferably 10 cm) to establish a "banana-shaped" or a "cigar-shaped" probability gradient of migrating photons. By changing the spacings, the optical module can target tissue at different depths and thus obtain three-dimensional optical data. Preferably, the optical module includes a plurality of symmetrical pairs of photon migration paths.

The described techniques can generate the amplitude cancellation or phase cancellation optical patterns, which demonstrate for single or multiple source-detector pairs remarkable sensitivity to small objects. Using back-projection algorithms or other imaging algorithms, it is possible to image a tissue region in less than a minute and with two dimensional resolutions of <1 cm in two dimensional displays. The present optical techniques can be used to examine internal tissue of an adult, child, neonate or in utero fetus and evaluate tissue functionality, physiology or pathological abnormality.

The methods of producing an image may include the steps of providing and using on the subject an imaging apparatus according to any of the foregoing aspects. In certain preferred embodiments, an optical contrast agent or a drug is introduced to the blood stream of the subject, and the apparatus is employed to produce image data sets of the examined tissue while the contrast agent or drug is present in blood or the tissue of the subject. The introduced contrast agent or drug may be preferentially absorbed in a localized tissue type or structure.

Other advantages and features of the invention will be apparent from the following description of the preferred embodiment and from the claims.

## Brief Description of the Drawings

Figs. 1, 1A and 1B show different optical modules located on the abdomen of a pregnant woman.

Fig. 1C is a cross-sectional view of the uterus of woman shown in Fig. 1 displaying light emitted from the optical modules shown in Fig. 1A or 1B.

Figs. 2, 2A and 2B show different optical modules located on the back of a subject for transabdominal or transthoracic examination.

Figs. 3 and 3A show diagrammatically respective single wavelength and dual wavelength phase cancellation imaging systems that employ the optical module of Fig. 1A or Fig. 1B.

Fig. 3B is a timing diagram used by the imaging system of Figs. 3 and 3A.

Figs. 4 and 4A show diagrammatically another embodiment of the phase cancellation imaging system employing the optical module of Fig. 1A.

Fig. 5 shows diagrammatically another embodiment of the phase cancellation imaging system employing the optical module of Fig. 1A.

Fig. 6 shows schematically an amplitude cancellation imaging system using another embodiment of the optical module shown in Fig. 6A.

Figs. 7, 7A and 7B show different embodiments of a cooling module used with a broad band light source such as a tungsten light bulb.

Fig. 8 shows diagrammatically another embodiment of the amplitude cancellation imaging system employing the optical module of Fig. 1B

Fig. 8A shows a circuit configuration for one element of the amplitude cancellation imaging system of Fig. 8.

Fig. 8B is a timing diagram used by the imaging system of Fig. 8.

Fig. 8C shows diagrammatically one channel of the amplitude cancellation imaging system of Fig. 8.

Fig. 8D shows diagrammatically another embodiment of the amplitude cancellation imaging system of Fig. 8.

Fig. 9 is an example of a "four-dimensional" graph for summarizing optical data and characterizing suspicious tissue structures.

## Description of the Preferred Embodiments

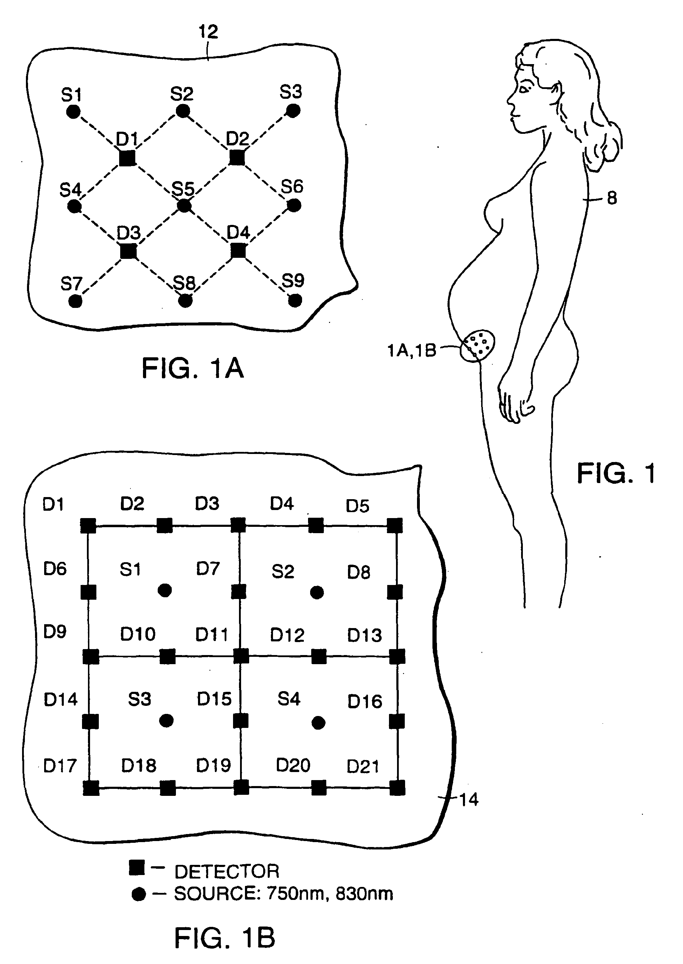

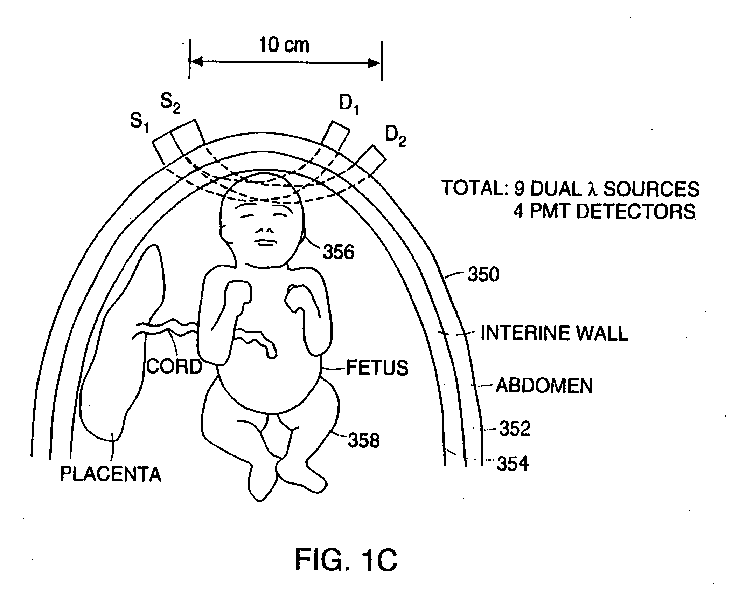

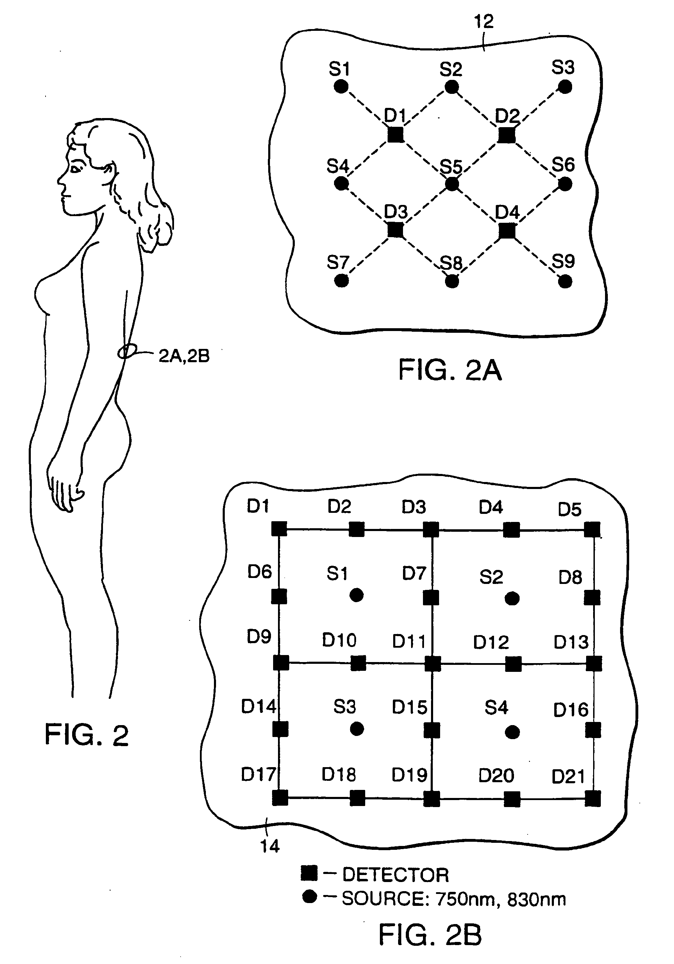

Referring to Figs. 1 through 2B, a selected fetal tissue of a fetus inside a female subject 8 is examined non-invasively using an imaging system connected to an optical module 12 or 14. Optical modules 12 and 14 include a multiplicity of light sources (e.g., laser diodes, LEDs, flashlight bulbs) providing light in the visible to infrared range and light detectors (e.g., photo multiplier tubes, Si diode detector, PIN, avalanche or other diode detectors), which may also include interference filters. The light sources and the light detectors are arranged to form selected geometrical patterns that provide a multiplicity of source-detector paths of photon migration inside the examined organ. The imaging system provides an *in vivo* image of the examined tissue. The image shows a location and size of an abnormal structure in the tissue, such as a tumor or bleeding. Furthermore, the image provides a qualitative and quantitative measure (e.g., metabolism, metabolic biochemistry, pathophysiology) of the abnormal structure. Alternatively, an optical module includes a multiplicity of optical fibers connected to one or several light sources, and a multiplicity of optical detection fibers connected to one or several light detectors as described in the PCT applications PCT/US96/00235 and PCT/US96/11630 (filed January 2, 1996 and July 12, 1996), both of which are incorporated by reference.

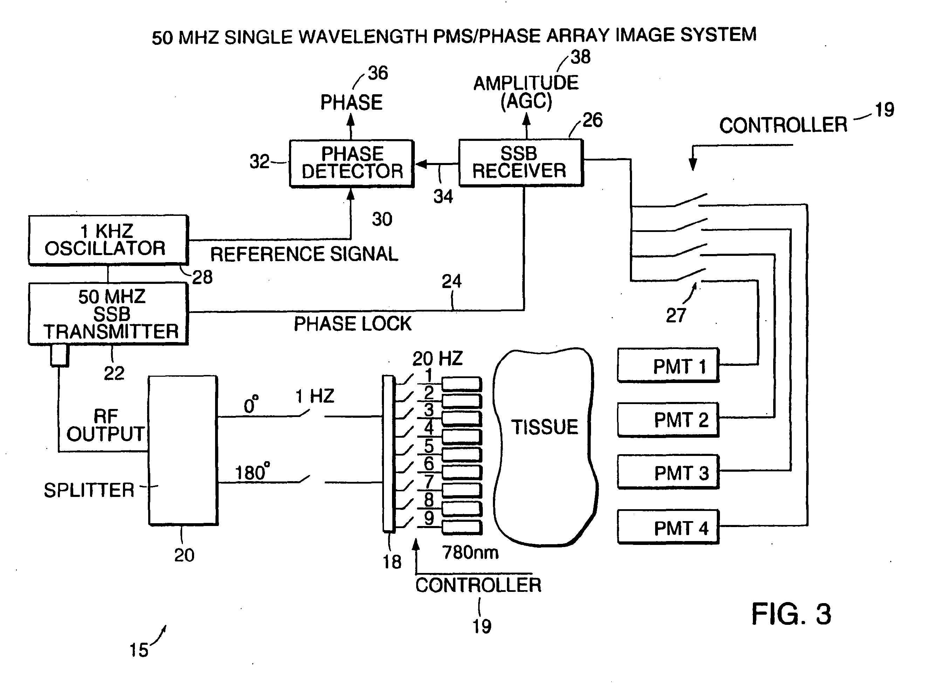

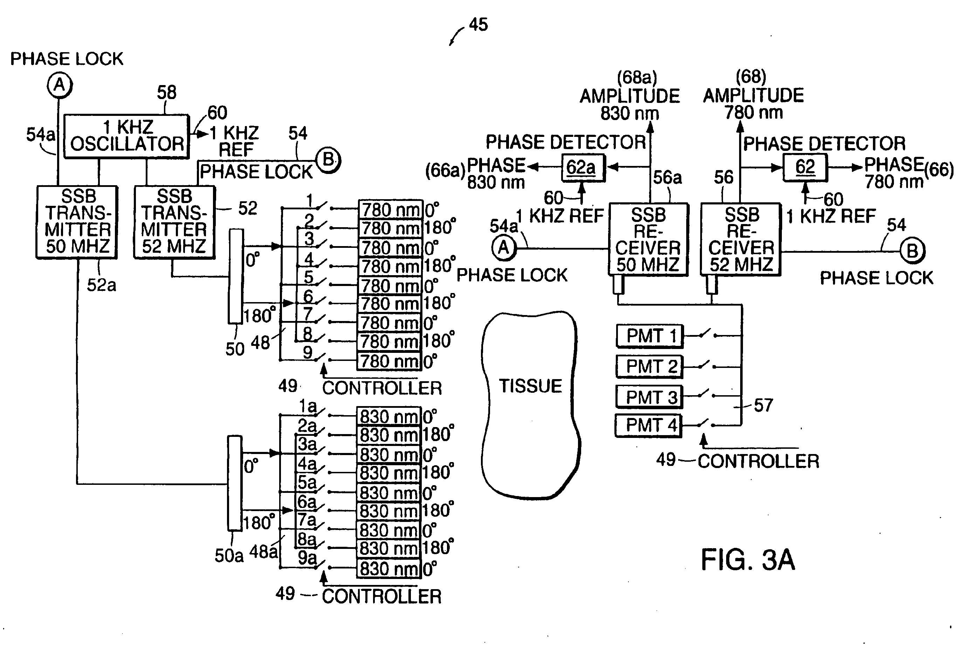

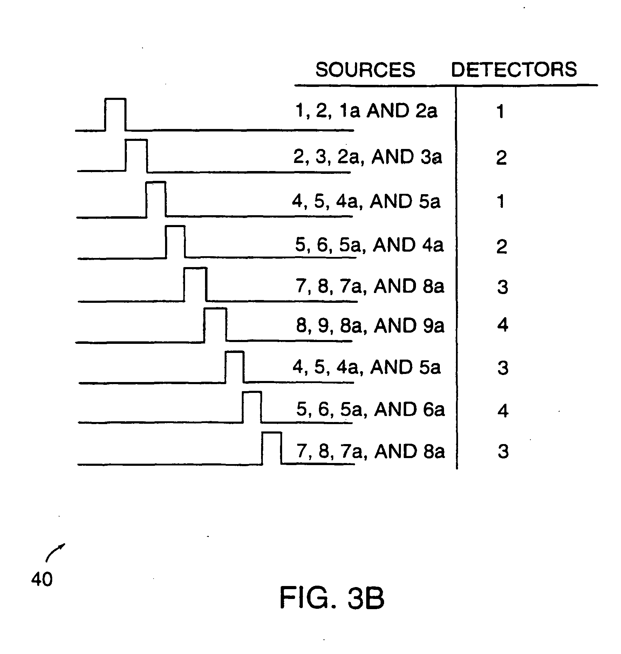

In one embodiment, optical module 12 includes nine laser diodes $S_1$, $S_2$,..., $S_9$ and four photo multiplier tubes (PMTs) $D_1$, $D_2$, $D_3$, $D_4$. The laser diodes and PMTs are embedded in a pliable rubber-like material positioned in contact with the scalp. There is a Saran (R) wrap or similar material located between the laser diodes and the skin, and between the PMTs and the skin. Similarly, optical module 14 includes four laser diodes $S_1$, $S_2$, $S_3$, $S_4$ and 27 silicon diode detectors $D_1$, $D_2$, ..., $D_{27}$ embedded in a pliable rubber-like material. The imaging systems shown in Figs. 3 through 7 may be interfaced with optical module 12 or 14 for imaging of the tissue. Furthermore, the imaging systems shown in Figs. 3 through 7, may be interfaced with two identical optical modules (12 or 14) located to probe symmetrical organs or tissue regions, such as the right kidney and the left kidney for lateralization, that is, comparative examination of the symmetric parts of the tissue. For calibration, the optical module may also be placed on one or several models having the same scattering coefficient and the same absorption coefficient as the normal tissue of the examined organ.

Referring to Figs. 1A and 3, a phased array imaging system 15 is connected to optical module 12 with nine laser diodes $S_1$, $S_2$,..., $S_9$ and four PMTs $D_1$, $D_2$, $D_3$, $D_4$ (e.g., Hamamatsu R928, Hamamatsu R1645u, TO8 1 cm$^2$ GaAs photomultiplier tube) powered by a high voltage supply (not shown). Four laser diodes surround each PMT forming an equidistant arrangement (for example, different optical modules may use distances of 3.5 cm, 7 cm, 10 cm, or 15 cm). A switch 18 connects laser diodes $S_1$, $S_2$,..., $S_9$ to a phase splitter 20, which provides to the diodes an RF modulation signal having both a 0 degree phase and a 180 degree phase. Imaging system 15 also includes a 50 MHz single side band transmitter 22 connected by a phase lock loop 24 to a 50 MHz single side band receiver 26. Single side band (SSB) transmitter 22 is connected to a 1kHz oscillator 28, which provides a reference signal 30 to a phase detector 32. SSB receiver 26 is connected to a switch 27, which connects one of the four PMTs (0.5 μV sensitivity) depending on control signals from a controller 19. The SSB transmitter-receiver pair can operate in the frequency region of 10-1000 MHz (preferably 50-450 MHz). The SSB receiver detects signal levels on the order of microvolts in a 2 KHz bandwidth. The phase noise of this apparatus is less than about 0.1°. This narrow bandwidth limits the spread of switching of various light sources to approximately 1.0 msec, and thus the sequencing time for an entire image of 16 source detector combinations can be ~1 sec. The system uses a 1 sec averaging time.

Controller 19, connected to a personal computer (not shown), sequences laser diodes $S_1$, $S_2$,..., $S_9$ so that two diodes receive 0° phase and 180° phase signals from splitter 20, every 0.1 sec. At the same time, controller 19 connects a symmetrically located PMT to SSB receiver 26. As shown in a timing diagram 40 (Fig. 3B), phased array imaging system 15 triggers two sources so that they emit modulated light of a 0° phase and a 180° phase for about 100 msec, and at the same time triggers a symmetrically located PMT. For example, when laser diodes 1 ($S_1$) and 2 ($S_2$) emit light of a 0° and 180° phase, respectively, and detector 1 ($D_1$) detects light that has migrated in the examined tissue. SSB receiver 26, which is phase locked with SSB transmitter 22, receives signal from detector 1 and provides output signal 34 to phase detector 32. Phase detector 32 measures the phase (36) of the detected light, and SSB receiver 26 provides the amplitude (38) of the detected light This phase detection circuit was described in U.S. Patent 4,972,331, which is incorporated by reference.

In the next cycle, controller 19 directs switch 18 to connects laser diodes 2 ($S_2$) and 3 ($S_3$), which emit modulated light of a 0° phase and a 180° phase, respectively, and detector 2 ($D_2$) detects light that has migrated in the examined tissue. Controller 19 also directs switch 27 to connect detector 2 to SSB receiver 26, which receives detection signal corresponding to the photons that have migrated from laser diodes 2 and 3 to detector 2. Again, phase detector 32 measures the phase (36) of the detected light, and SSB receiver 26 provides the amplitude (38) of the detected light. The duration of each pair of light flashes is 100 msec. The complete set of data for all source - detector combinations is collected every 30 sec. A computer (not shown) stores the phase values and the amplitude values measured for the different combinations shown in timing diagram 40 and employs these values to create images of the examined tissue, as is described below. The computer uses the ADA2210 board for data acquisition.

Before or after the above-described measurement, phased array imaging system 15 may be calibrated on a model of the uterus and the fetus. The in utero fetal model includes a large vessel that models the uterus and a smaller chamber that models the fetal head. This chamber can be placed several centimeters deep as is the fetal head beneath the abdominal and uterine layers. The fetal head chamber is filled with Intralipid® (scatterer) and human blood (absorber), and the large vessel with amniotic fluid (water). The model is constructed to change in blood oxygenation and blood volume using a tubing connected to the chamber. The calibration includes a variety of fetal conditions over a range of blood oxygenation and volume values of the fetal brain as well as a range of optical properties and thickness of the uterine and abdominal tissue layers.

During the calibration procedure, the optical module is placed on the model, and the imaging system collects the phase data and the amplitude data using the sequences shown in the timing diagram 40. The scattering coefficient and the absorption coefficient of different types of tissue can be measured as described in U.S. Patent 5,402,778. Furthermore, the optical signals are measured as a function of change in the position of the fetal head to determine the signal displacement as a function of fetal head position. The sensitivity and detection limit is a function of the blood oxygenation and volume of the fetal brain, the maternal tissues and the various positions of the fetal head with respect to the source and detector for both the short and long source-detector separations (i.e., the relative volume of the fetal tissue and amniotic fluid). The phased array system has a very high positional accuracy and object detection at a depth of several centimeters inside the model.

Phased array imaging system 15 generates a "model" image for each wavelength employed. The model image may later be subtracted from the tissue images to calibrate the system and also account for the boundary conditions of the light migrating in the tissue. Alternatively, phased array imaging system 15 is calibrated prior to taking measurement data and the gain on the light sources or the detectors is adjusted to obtain selected values.

Referring to Figs. 1A and 3A, a dual wavelength phased array imaging system 45 is connected to optical module 12 with nine 780 nm laser diodes $S_1$, $S_2$,..., $S_9$, nine 830 nm laser diodes $S_{1a}$, $S_{2a}$,..., $S_{9a}$, and the four PMTs $D_1$, $D_2$, $D_3$, and $D_4$ powered by a high voltage supply (not shown). Pairs of laser diodes $S_1$ and $S_{1a}$, $S_2$ and $S_{2a}$, ..., $S_9$ and $S_{9a}$ are located next to each other and arranged to introduce modulated light at almost the same tissue locations. A switch 48 connects laser diodes $S_1$, $S_2$, ... $S_9$ to a phase splitter 50, which provides to the laser diodes an RF modulation signal having both a 0 degree phase and a 180 degree phase. Similarly, a switch 48a connects laser diodes $S_{1a}$, $S_{2a}$,..., $S_{9a}$ to a phase splitter 50a, which provides to the laser diodes an RF modulation signal having both a 0 degree phase and a 180 degree phase. A 52 MHz SSB transmitter 52 is connected by a phase lock loop 54 to a 52 MHz SSB receiver 56, and a 50 MHz SSB transmitter 52a is connected by a phase lock loop 54a to a 50 MHz SSB receiver 56a. Both SSB transmitters 52 and 52a are connected to a 1kHz oscillator 58, which provides a reference signal 60 to phase detectors 62 and 62a. SSB receivers 56 and 56a are connected one of the four PMTs by a switch 57 depending on control signals from controller 49. Controller 49, connected to a personal computer, sequences the laser diodes so that two pairs of the laser diodes receive 0° phase and 180° phase signals from splitters 50 and 50a, and at the same time controller 49 connects a symmetrically located detector to SSB receivers 56 and 56a.

As shown in timing diagram 40 (Fig. 3B), phased array imaging system 45 triggers for each wavelength two sources that emit simultaneously modulated light of a 0° phase and a 180° phase for about 100 msec and, at the same time, controller 49 connects the symmetrically located PMT. For example, switch 48 connects SSB transmitter 52 to 780 nm laser diode 4 ($S_4$) to emit 52 MHz modulated light of a 180° phase and connects 780 nm laser diode 5 ($S_5$) to emit 52 MHz modulated light of a 0° phase. At the same time, switch 48a connects SSB transmitter 52a to 830 nm laser diode 4a ($S_{4a}$) to emit 50 MHz modulated light of a 180° phase and connects 830 nm laser diode 5a ($S_{5a}$) to emit 52 MHz modulated light of a 0° phase. Simultaneously, switch 57 connects detector 1 ($D_1$) to SSB receivers 56 and 56a to receive the detection signal corresponding to photons of both wavelengths that have migrated in the examined tissue. Phase detector 62 provides the phase (66) of the detected 780 nm light, and phase detector 62a provides the phase (66a) of the detected 830 nm light for the selected geometry. Similarly, SSB receiver 56 measures the amplitude (68) of the detected 780 nm light and SSB receiver 56a measures the amplitude (68a) of the detected 830 nm light. This operation is repeated for all combinations of sources and detectors shown in timing diagram 40. A computer (not shown) stores the phase value and the amplitude value measured for the different combinations shown in timing diagram 40. The computer then uses the measured values to create images using appropriate algorithms.

Several phased array systems were described in the PCT application PCT/US 93/05868 (published as WO 93/2514 on December 23, 1993). This PCT publication also describes the basic principles of phase and amplitude cancellation. The phased array imaging system uses a detector for detecting light emitted from equidistant sources located symmetrically with respect to the detector (or one source and several equidistant detectors located symmetrically). If two sources $S_1$ and $S_2$ emit modulated light having equal amplitude and a 0° phase and a 180° phase, detector $D_1$ located in the middle detects a null in the amplitude signal and detects a crossover between the 0° and 180° phase, i.e., a 90° phase, for substantially homogeneous tissue. That is, the detector is located on the null plane. In heterogeneous tissue, the null plane is displaced from the geometric midline. Nevertheless, the null establishes an extremely sensitive measure to perturbation by an absorber or scattered. Furthermore, at the null condition, the system is relatively insensitive to amplitude fluctuations common to both light sources, and insensitive to inhomogeneities that affect a large tissue. The system has a high sensitivity to scattering provided that the scattering contrast is the same as the absorbing contrast. The system can readily observe shifts of 50 to 60° of phase under altered blood volume or blood oxygenation conditions, where the phase noise is less than a 0.1° (s/n >400) for a 1 Hz bandwidth. The amplitude signal is little less useful in imaging since the position indication is somewhat ambiguous, i.e., an increase of signal is observed regardless of the displacement of the absorbing object with respect to the null plane, although this is remedied by further encoding of the sources.

As described in the PCT application PCT/US 93/05868, the light sources excite a photon diffusion wave, due to cancellation effects, that has a relatively long wavelength (~10 cm), determined by the scattering ($\mu_s'$ = 10 cm$^{-1}$) and absorption ($\mu_a$ = 0.04 cm$^{-1}$) properties of the tissue. The photon diffusion wavelength of about 10 cm provides imaging in the "near field." The imaging system may use light sources of one or several optical wavelengths in the visible to infrared range, depending on the characteristic to be imaged (i.e., blood volume, blood oxygenation, a distribution of a contrast agent in the tissue, an absorbing constituent of the tissue, a fluorescing constituent of the tissue, or other). The phase signal at zero crossing detection is essentially a square wave "overloaded" signal. It is moderately insensitive to the changes of signal amplitude that may occur in imaging from proximal to distal source-detector pairs and is also moderately insensitive to ambient light.

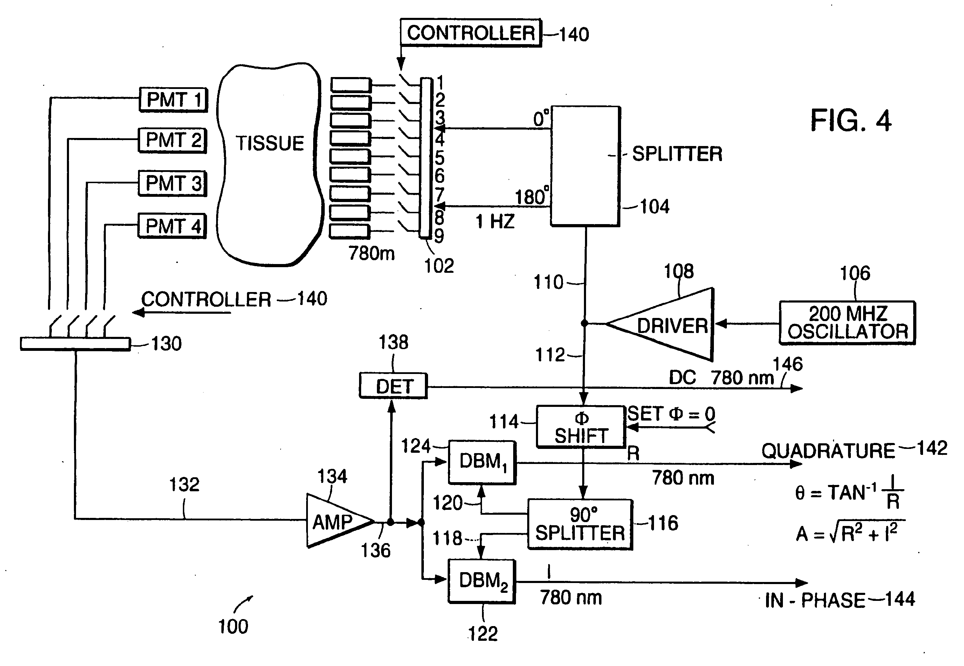

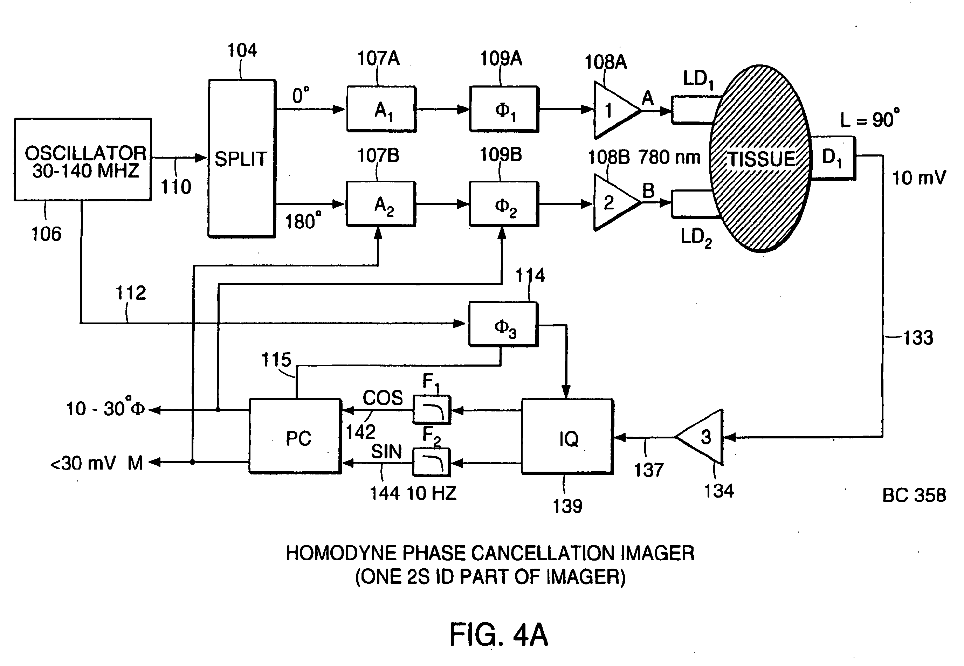

Referring to Fig. 4, in another embodiment, a phased array imaging system 100 is used instead of imaging systems 15 or 45. Imaging system 100, connected to optical module 12 (shown in Fig. 1A) having nine laser diodes $S_1$, $S_2$,..., $S_9$ and four PMTs $D_1$, $D_2$, $D_3$, and $D_4$, employs homodyne phase detection. A switch 102 connects laser diodes $S_1$, $S_2$,..., $S_9$ to a phase splitter 104, which provides to the diodes an RF modulation signal having both a 0 degree phase and a 180 degree phase. Imaging system 100 also includes a 200 MHz oscillator 106 providing RF signal to a driver 108, which is connected to phase splitter 104. (Alternatively, an oscillator in the range of 10 - 1000 MHz, preferably 50 - 500 MHz, may be used.) A phase shifter 114 receives the drive signal (112) from driver 108 and provides the signal of a selected phase (e.g., a 0° phase change) to a 90° phase splitter 116. Phase splitter 116 provides a 0° phase signal (118) and a 90° phase signal (120) to double balance mixers (DBM) 122 and 124, respectively.

A controller 140, connected to a personal computer (PC), sequences laser diodes $S_1$, $S_2$,..., $S_9$ using switch 102 so that two diodes receive modulate signal at a 0° phase and a 180° phase from splitter 104. At the same time, a controller 140 connects a symmetrically located PMT using a switch 130 to an amplifier 134. Amplifier 134 provides a detection signal (136) to double balance mixers 122 and 124, and to a DC detector 138. Double balance mixer 122 receives the detection signal (136) and the 0° phase reference signal (118) and provides an in-phase signal I (144). Double balance mixer 124 receives the detection signal (136) and the 90° phase reference signal (120) and provides a quadrature signal R (142). DC detector 138 provides DC signal (146). The in-phase signal I and quadrature signal R specify the phase ($\theta = \tan^{-1} I/R$) of the detected optical radiation and the amplitude ($A = (R^2 + I^2)^{-\frac{1}{2}}$) of the detected optical radiation. This phase detection circuit was described in U.S. Patent 5,553,614.

Similarly as for imaging systems 15 and 45, imaging system 100 directs controller 140 to sequence the laser diodes and the PMT detectors using timing diagram 40. The computer stores the phase value and the amplitude value measured for each of the combinations and generates images described below.

Fig. 4A shows diagrammatically one portion of phase cancellation, phased array imaging system 100. The depicted portion of imaging system 100 includes two laser diodes $LD_1$, and $LD_2$ and a light detector $D_1$, which are included in optical module 12 or 14. Oscillator 106 provides carrier waveform having a frequency in range of 30 to 140 MHz. The carrier waveform frequency is selected depending on the operation of the system. When time multiplexing the light sources using switch 102, then the carrier waveform is modulated at a lower frequency, e.g., 30 MHz to afford switching time.

When no time multiplexing is performed, oscillator 106 operates in the 100 MHz region. Splitter 104 splits the oscillator waveform into 0° and 180° signals that are then attenuated by digitally controlled attenuators 107A and 107B by 0% to 10% in amplitude. The phase of the attenuated signals is appropriately shifted by digitally controlled phase shifters 109A and 109B in the range of 10° - 30° and preferably 20° in phase. Laser drivers 108A and 108B drive $LD_1$ and $LD_2$, respectively, which emit light of the same wavelength, for example, 780 or 800 nm. After the introduced light migrates in the examined tissued, a PMT detector $D_1$ amplifies the detected signals having initially the 0 and 180° phases. As described above, for homogeneous tissue and symmetric locations of $LD_1$, $LD_2$ and $D_1$, the output of the PMT is 90°, i.e., halfway between 0° and 180° and the amplitude is close to zero. The personal computer (PC) adjusts the attenuation provided by attenuator 107B and the phase shift provided by phase shifter 109B so that detector $D_1$ detects phase nominally around 25° and amplitude nominally around s 10 millivolts for homogeneous tissue. This signal is connected to amplifier 134 and to the IQ circuit 139. The cosine and sine signals are fed into the personal computer, which takes the amplitude (the square root of the sum of the squares of I and Q) and the phase angle (the angle whose tangent is I/Q) to give outputs of phase around 25° and amplitude signals around 10 millivolts. The personal computer also adjusts the reference signal to the IQ to have the phase $\phi_3$ between 10° to 30° and preferably around 25°, i.e., phase shifter 114 provides to the IQ circuit 139 the reference phase having a value selected by the combination of phase shifters 109A and 109B.

In a currently preferred embodiment, splitter 104 is a two way 180° power splitter model number ZSCJ-2 1, available from Mini-Circuits (P.O. Box 350186, Brooklyn, New York 11235-0003). The phase shifters 109A, 109B and 114 and attenuators 107A, and 107B are also available from Mini-Circuits, wherein the attenuators can be high isolation amplifier MAN-1AD. IQ demodulator 139 is a demodulator MIQY-140D also available from Mini-Circuits.

The system obtains the initial values of attenuator 107B ($A_2$) and phase shifter 109B ($\phi_2$) on a model or a symmetric tissue region (e.g., the contralateral kidney or another region of the same organ that is tumor free). The entire probe is calibrated on a tissue model by storing the calibration values of $A_2$ and $\phi_2$ for the various source-detector combinations (i.e., the baseline image). The probe is then moved to the abdomen, for example, and the phases and amplitudes are detected for the various source and detector combinations. When the contralateral tumor free kidney is used as a model, the probe is transferred to the contralateral kidney (taking note to rotate the probe because of the mirror image nature of the kidney physiology) and then the images are read out from all the source-detector combinations to acquire the tissue image.

There is no limitation on multiplexing as long as the bandwidth of $F_1$ and $F_2$ is recognized as being the limiting condition in the system normalization. It should be noted that normalization must be accurate and without "dither" and therefore, a significant amount of filtering in F, and $F_2$, i.e., less than 10 Hz bandwidth. If $\phi_2$ is adjusted over a large range, there will be an amplitude-phase crosstalk. Thus, the system may adjust phase and then amplitude and repeat these adjustments iteratively because of the amplitude phase crosstalk. The control of $A_1$ and $\phi_1$ provides even a greater range of control, where obviously inverse signals would be applied to them, i.e., as the $A_1\phi_1$ signals are increased, the $A_2$, $\phi_2$ signals would be decreased. Both $A_2$ and $\phi_2$ can be controlled by PIN diodes, to achieve an extremely wideband frequency range. However, since signal processing controls the bandwidth of the feedback system, that either PIN diode or relay control of the phase and amplitude is feasible for automatic compensation. If, in addition, dual wavelength or triple wavelength sources are used, each one of them must be separately calibrated because no two light sources can be in the same position relative to the imaged tissue (unless, of course, they are combined with optical fibers).

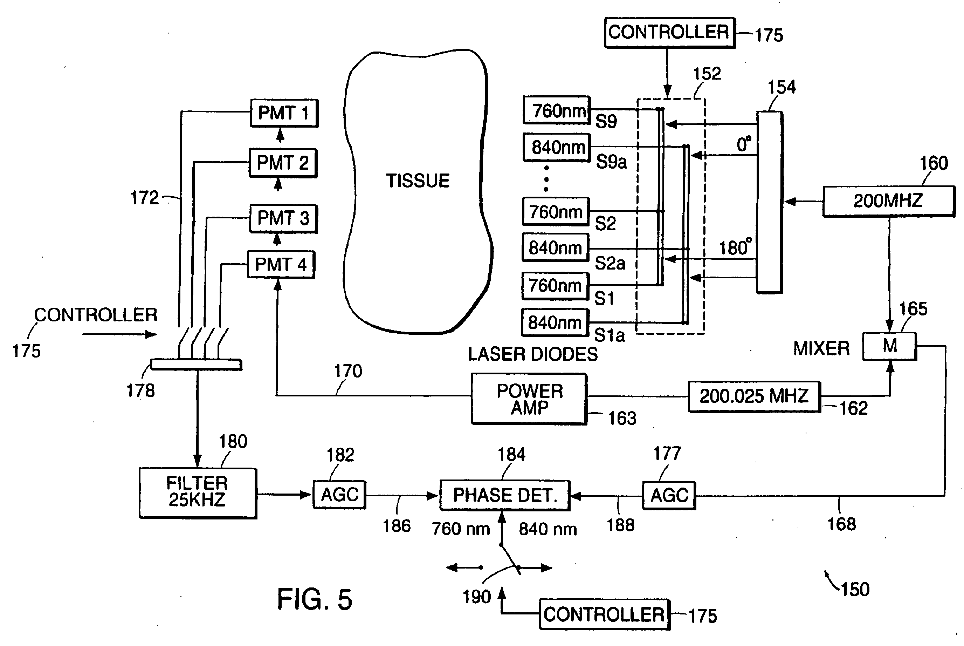

Referring to Fig. 5, in another embodiment, a dual wavelength phased array optical system 150 is used instead of optical systems 15, 45 or 100. Optical system 150, connected to optical module 12 (shown in Fig. 1A) having nine 760 nm laser diodes $S_1$, $S_2$,..., $S_9$, nine 840 nm laser diodes $S_{1a}$, $S_{2a}$,..., $S_{9a}$ and four PMTs $D_1$, $D_2$, $D_3$, and $D_4$ is based on heterodyne phase detection. A switch 152 connects the laser diodes to a phase splitter 154, which provides to the diodes an RF modulation signal having both a 0 degree phase and a 180 degree phase. Imaging system 150 employs a mixer 165 connected to a 200 MHz oscillator 160 and 200.025 MHz oscillator 162 (Alternatively, oscillators operating in the range of 10 - 1000 MHz, preferably 50 - 500 MHz, may be used.) Mixer 165 provides a 25 kHz reference signal (168) to an adjustable gain controller 177. Oscillator 162 connected to power amplifier 163 provides a 200.025 MHz reference signal (170) to the second dynode of each PMT detector for heterodyne detection. Each PMT detector provides a 25 kHz detection signal (172) to a switch 178, which in turn provides the signal to a 25 kHz filter 180. A phase detector 184 is connected to an adjustable gain controller 182, which provides a filtered and amplified detection signal (186) and to adjustable gain controller 177, which provides the reference signal (188). Phase detector 184, connected to a switch 190, provides the detected phase value for each wavelength. This phase detection circuit was described in U.S. Patent 5,187,672. Another type of phase detection circuit was described in U.S. Patent 5,564,4,17, which is incorporated by reference.

Similarly as described above, controller 175, connected to a personal computer, sequences laser diodes $S_1$, $S_2$, $S_9$ or laser diodes $S_{1a}$, $S_{2a}$,..., $S_{9a}$ using switch 152 so that two diodes emitting the same wavelength receive 0° phase and 180° phase signals from splitter 154. At the same time, controller 175 connects a symmetrically located PMT using a switch 178 to filter 180 and adjustable gain controller 182. Phase detector 184 provides the measured phase. Imaging system employs timing diagram 40 (Fig. 3B); however, since the two wavelength light is not frequency encoded, laser diodes $S_1$, $S_2$,..., $S_9$ or laser diodes $S_{1a}$, $S_{2a}$,..., $S_{9a}$ are triggered in each sequence. That is, light of only one wavelength is detected in each cycle. For each wavelength, the computer stores the phase values measured for the different combinations. The computer also generates images described below.

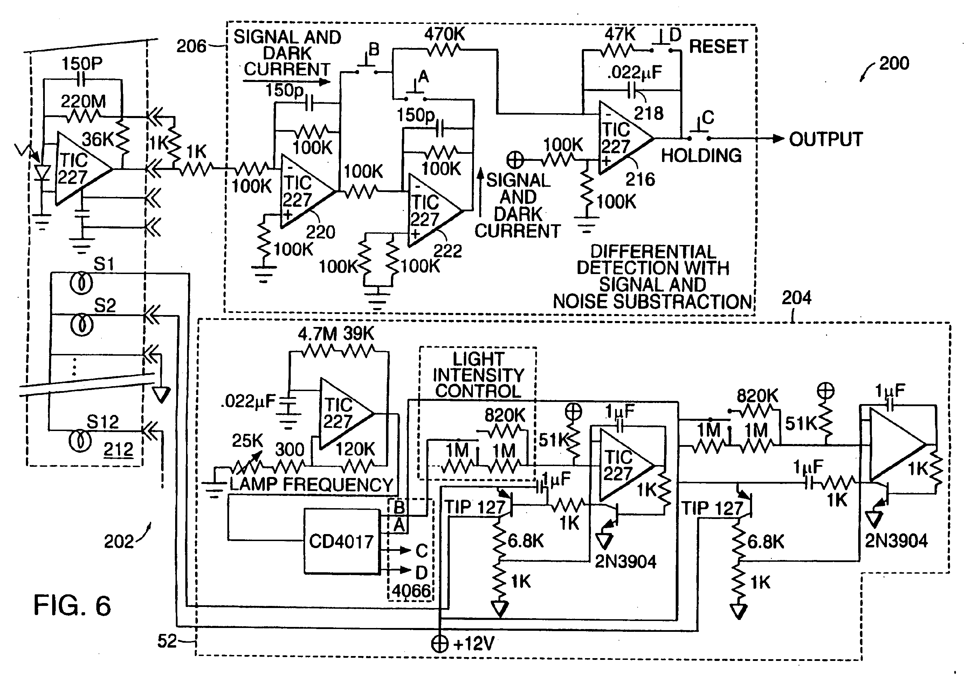

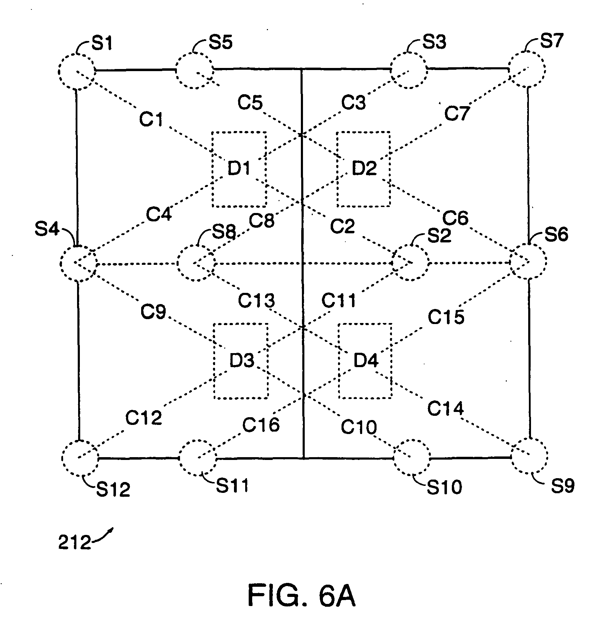

Referring to Fig. 6, in another embodiment, an amplitude cancellation imaging system 200 uses an optical module 212 shown in Fig. 6B. Optical module 212 includes twelve light sources S1, S2, ..., S12 and four light detectors D1, D2, D3, and D4 mounted on a plastic or rubber foam material. The light sources and the light detectors are located on a geometrical pattern that provides sixteen source-detector combinations (C1, C2, ..., C16) having a selected source-detector separation. The separation may be 2.5 cm to produce about 1.25 cm average light penetration. (Several modules with different source-detector separations may be used to obtain several two dimensional images of different tissue depths. Alternatively, a single module - may include source detector combinations providing different separations. The light penetration depth is approximately one half of the source-detector separation.) The light sources are 1 W tungsten light bulbs, which emit broad band non-modulated light. The light detectors are silicon diodes, each equipped with an interference filter transmitting a 10 nm wide band centered at 760 nm and 850 nm. The 760 nm and 850 nm wavelengths are selected to detect oxyhemoglobin and deoxyhemoglobin in the examined tissue.

Optical module 212 is connected to an analog circuit 202, which includes a source circuit 204 for controlling sources S1, S2, ... S 12. Optical module 212 is connected to a detector circuit 206, which controls diode detectors D1, D2, D3 and D4. In general, imaging system 200 can turn ON each source for a selected period in the range of $10^{-6}$ sec. to 0.1 sec., and one or several symmetrically located detectors are turned on simultaneously or sequentially to collect optical data. Specifically, one of sources S1, S2, ... S12 is turned ON for 500 msec and the emitted light is introduced into the tissue from the corresponding input port. The introduced photons migrate over banana shaped paths in the examined tissue to a detection port. The corresponding detector is triggered 200 msec after the source and collects light for 200 msec. Detector circuit 206 receives a detector signal from the diode detector. Detection circuit 206 enables correction for the dark current/noise that comprises background light, DC offset of the operational amplifiers, photodiode dark current, temperature effects on the outputs of individual components and variations due to changing environment.

Imaging system 200 performs data acquisition in four steps synchronized by its internal oscillator. The first step is performed by having the light sources OFF. The detector output is directed to an integrator 216 and integration capacitor 218 is charged to the dark level voltage. In the second step, the light source is turned ON and after 200 msec the preamplifier output that corresponds to the intensity of the detected light is directed to integrator 216 in a way to charge capacitor 218 with current of polarity opposite to the polarity of the charging current in the first step. This is achieved using an appropriate ON/OFF combination of switches A and B. The voltage of capacitor 218 is charging to a value that, after 200 msec., represents the total detected intensity minus the dark level noise signal. In the third step, both switches A and B are turned OFF to disconnect both the positive unity gain and the negative unity gain operational amplifiers (220 and 222). Then, the output of integrator 218 is moved via switch C to an analog-to-digital converter and the digital signal is stored in the memory of a computer. In the fourth step, the switches A, B and C are open and switch D is closed in order to discharge capacitor 218 through a 47K resistor. At this point, the circuit of integrator 216 is reset to zero and ready for the first step of the detection cycle.

Alternatively, analog circuit 202 may be replaced by a computer with an analog-to-digital converter and appropriate software that controls the entire operation of optical module 212. The computer controls the sources and the detectors of optical module 212 in a similar way as described above. The detected dark level noise signal is digitally subtracted from the detected intensity of the introduced light. The collected data sets are processed using an imaging algorithm. The imaging algorithm calculates the blood volume of the examined tissue for each source-detector combination for each data set. The imaging algorithm can also calculate the oxygenation of the examined tissue for each source-detector combination.

The blood volume or oxygenation images can be subtracted from "model" images. The blood volume image can be subtracted from the oxygenation image to create congruence data (further described below) to localize and characterize a tissue anomaly. The imaging algorithm may also create an image using the differential image data sets. Prior to creating the image, an interpolation algorithm is employed to expand the differential image data set, containing 16 (4x4) data points, to an imaging data set containing 32x32 image points.

Alternatively, the computer uses a back projection algorithm known in computed tomography (CT) modified for light diffusion and refraction and the banana like geometry employed by the optical imaging system. In the optical back projection algorithm, the probabilistic concept of the "photon migration density" replaces the linear relationship of ballistically transmitted X-rays, for the beam representing pixels. The photon migration density denotes a probability that a photon introduced at the input port will occupy a specific pixel and reach the detection port. For different types of tissue, the phase modulation spectrophotometer provides the values of the scattering and absorption coefficients employed in the probability calculations. (These values are determined as described in U.S. Patent 5,402,778.) In the image reconstruction program, the probability is translated into a weight factor, when it is used to process back projection. The back projection averages out the values of information that each beam carries with the weighting in each pixel. The specific algorithms are provided in U.S. Pat. 5,853,370 issued on Dec. 29, 1998.

A method for correcting blurring and refraction used in the back projection algorithm was described by S.B. Colak, H.Schomberg, G.W.'t Hooft, M.B. van der Mark on March 12, 1996, in "Optical Back projection Tomography in Heterogeneous Diffusive Media" which is incorporated by reference as if fully set forth herein. The references cited in this publication provide further information about the optical back projection tomography.

Referring to Fig. 6, in another embodiment, amplitude cancellation imaging system 200 uses optical module 14 shown in Fig. 6A. In this arrangement, four centrally located light sources S1, S2, S3, and S4 and 21 detectors D1, D2, ..., D21 provide a multiplicity of symmetric photon migration paths for each source. For example, source S1 is turned ON for a period in the range of $10^{-6}$ sec to 0.1 sec. The source emits non-modulated light into the examined tissue. Symmetrically located detectors D1 and D11 are ON simultaneously to collect introduced photons migrating over substantially symmetric paths. For symmetrical tissue conditions, detectors D1 and D11 detect light of the same intensity, and thus the differential signal is zero, i.e., the detected amplitudes are canceled. Imaging system 200 collects the differential data for a multiplicity of symmetric photon migration paths and generates an image of the examined tissue. Imaging system 200 may collect optical data for several wavelengths and generate blood volume images and blood oxygenation images for the examined tissue. Amplitude cancellation imaging system 200 may also use a second identical optical module 14 placed to examine a symmetrical tissue region, or a symmetrical organ, for example, the two modules may be positioned to examine the right and left lungs. The blood volume images or the blood oxygenation images collected for the two symmetric tissue regions may be subtracted to provide a differential image, which will further emphasize a tissue abnormality located in one tissue region.

Alternatively, the amplitude cancellation imaging system uses light modulated at frequencies in the range of 0.1 kHz to 100 kHz. The system employs the above-described algorithm, but the light sources emit frequency modulated light and the detectors, each connected to a lock-in amplifier, detect light modulated at the same frequency. This lock-in detection may further increase the signal to noise ratio by eliminating external noise. The detected light intensities are processed the same way as described above to image the examined tissue.

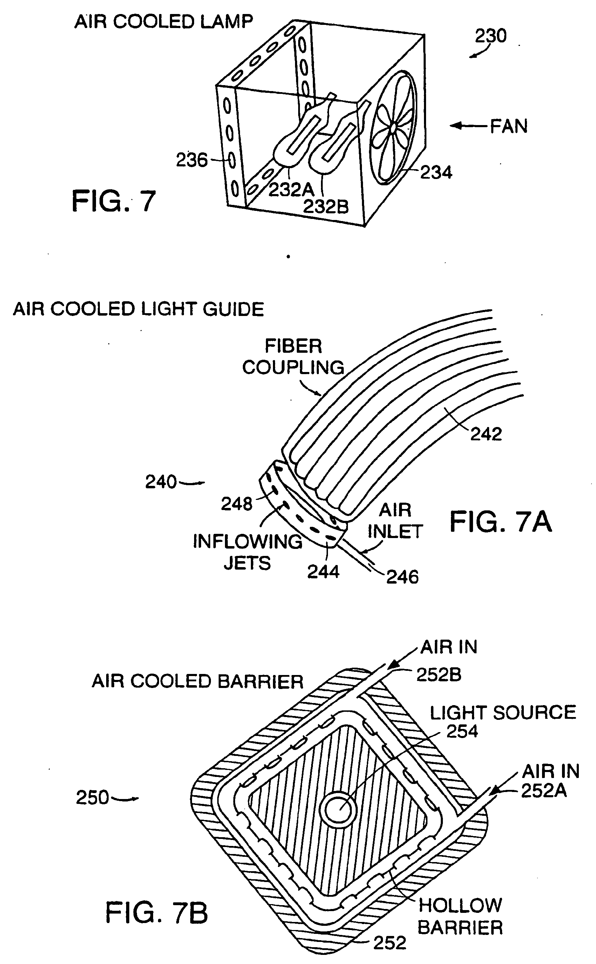

Figs. 7, 7A and 7B show different embodiments of a cooling module used with a broad band light source or light guides, where these are positioned close to the skin. The broad band light sources or light guides may create heat trapped close to the skin and thus uncomfortable temperature. Fig. 7 depicts a cooling module 230, which surrounds light sources 232A and 232B. Cooling module 230 includes a fan 234 and a set of air passages 236. In a similar design, two fans are juxtaposed on each side of one or more light bulbs to form an "open frame" so t6at - the fans blow not only upon the light sources, but upon the skin itself. The cooling module enables a power increase on the light sources, but no increase of heat upon the skin itself, which remains under comfortable conditions.

Fig. 7A depicts a cooling module 240 for cooling light guides. Light guides 242 deliver light and heat to the skin. A cooling ring 244 includes an air inlet 246 and a set of air passages 248 (or jets) for providing air flow to the irradiation location. Fig. 7B depicts a cooling module 250 constructed to air cool a light barrier 252. Light barrier 252 has similar optical properties as the light barrier described in the PCT application PCT/TJS92/04153 (published on November 26, 1992 as WO 92/20273). This embodiment utilizes the advantages of the light barrier and enables the use of higher light intensities. Cooling module 250 includes air inlets 252A and 252B, which provide air to a set of conduits and openings that deliver air to the skin near light source 254. Compressed air may also be used.

The safety regulations for delivering continuous otherwise non-coherent light of high intensities to the skin often depend on the temperature rise of the skin itself. For examination of large tissue volumes or deep tissues (i.e., where there is a large separation between the optical input and optical detection ports) relatively large light intensities are needed. Under conditions of prolonged even low level illumination, the skin may become uncomfortably warm and may blister. However, the erythemic effects are much smaller in the NIR, where the delivered heat is a factor, than they are in UVA and UVB, where cancer-producing damage may occur (but is not known for the NIR). The effect of the cooling air is not just convection of warm air away from the skin, but it enhances the evaporation of perspiration from the skin. Thus, as soon as the skin temperature rises and perspiration is initiated, greatly enhanced cooling is obtained with the forced air increasing the evaporation.

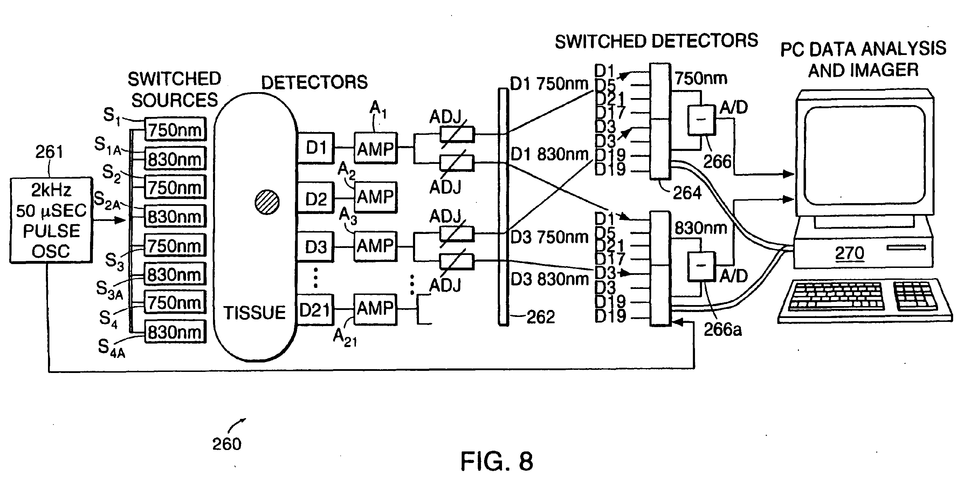

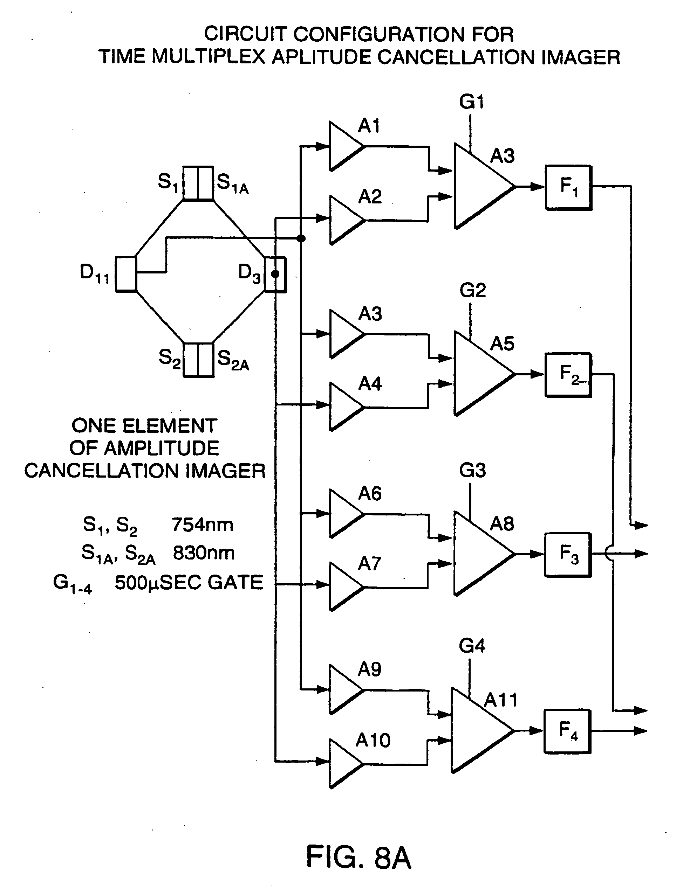

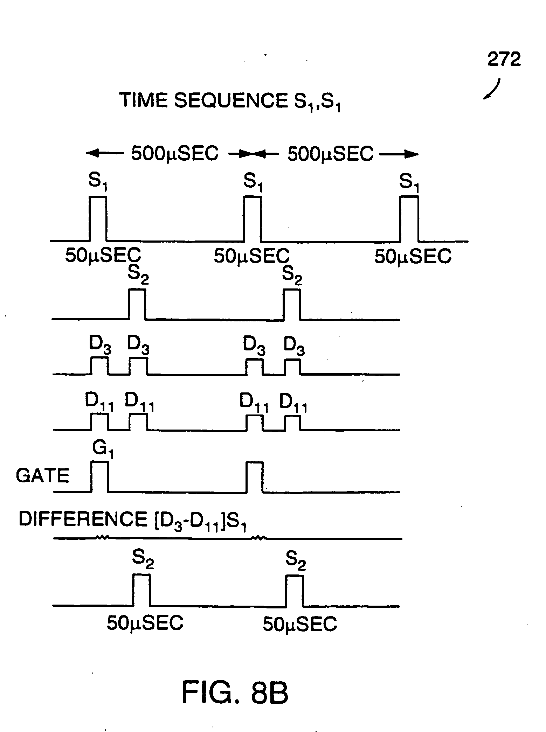

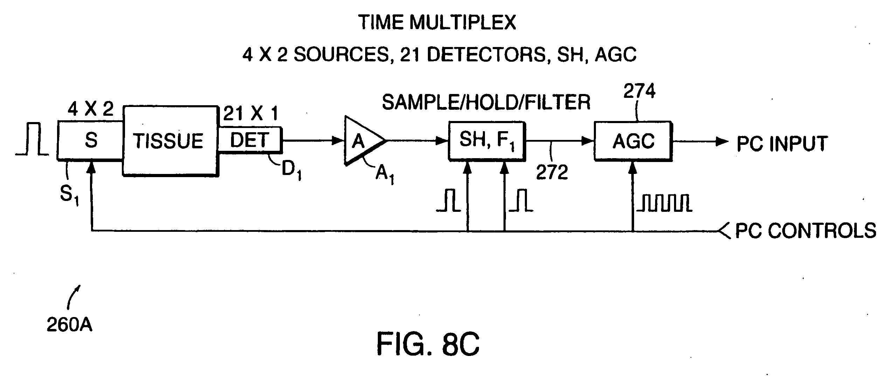

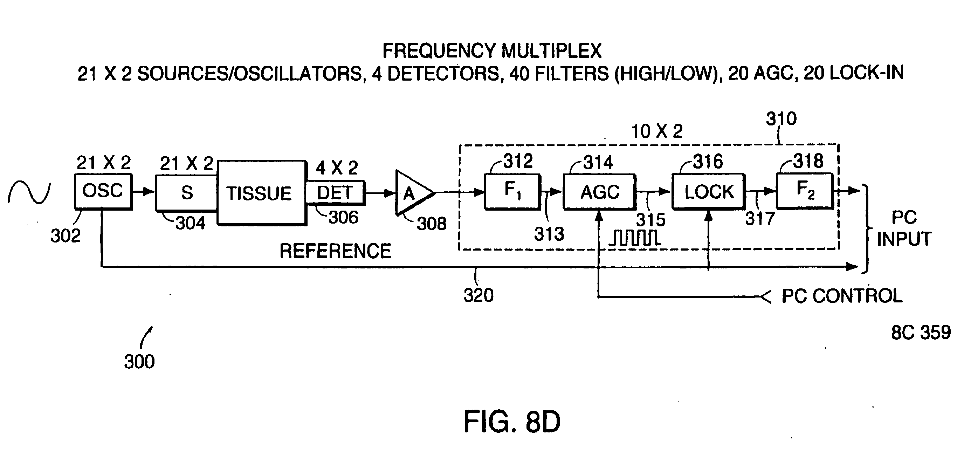

Referring to Fig. 8, an amplitude cancellation imaging system 260 is used instead of optical systems 15,45,100, 150, or 202. Dual wavelength amplitude cancellation imaging system 260 is connected to optical module 14, shown in Figs. 1B and 2B, which now incudes four 750 nm laser diodes $S_1$, $S_2$, $S_3$, and $S_4$, four 830 nm laser diodes $S_{1a}$, $S_{2a}$, $S_{3a}$, and $S_{4a}$, and twency-one silicon diode detectors $D_1$, $D_2$,..., $D_{21}$. Each detector is connected to a preamplifier and an adjustable gain controller that may be used initially for calibration. The detector outputs are switched by a switch 262 and a controller 264 so that analog-to-digital converters 266 and 266a receive 750 nm and 830 nm data, respectively, from two symmetrically located detectors. A computer 270 stores the detected values measured for the different combinations. The computer also generates images described below. Another type of amplitude detection circuit was described in Figures 1 through 13 and the corresponding specification of U.S. Patent 5,673.701.

Also referring to Figs. 8A and 8B, the controller sequences an oscillator 261 so that each source emits a 50 $\mu$sec light pulse as shown in timing diagram 272.. The system sequences through the various source/detector combinations in approximately one msec, and averages the imaged data over 8 sec to get a very high signal to noise ratio. Fig. 8A. shows the circuit configuration for one element of imaging system 260, i.e., 754 nm sources $S_1$, $S_2$ and 830 nm sources $S_{1a}$, $S_2$, and two symmetrically positioned detectors $D_3$ and $D_{11}$, also shown in Fig. 2A. The light intensities detected for the symmetrical locations are subtracted in a digital or analog way. The computer stores all differential data, detected for the two wavelengths, for generating tissue images.

Fig. 8C shows diagrammatically a single channel 260A of the time multiplex imaging system 260. Detector $D_1$ detects light emitted from light source $S_1$ emitting light pulses of the duration of about 50 $\mu$sec. The detector signal is amplified and provided to a sample-and-hold circuit and filter. Detector $D_1$ is a silicon diode detector that has the detection area of about 4 X 4 mm and includes a pre-amplifier. The filtered signal 272 is provided to an AGC 274, which adjusts the amplitude of the signal based on a control signal from a personal computer. The personal computer has normalization amplitudes for the individual source-detector combinations.

Amplitude cancellation imaging system 260 is normalized on a tissue model by detecting signals for the individual source-detector combinations and appropriately normalizing the detected signal using the AGC control. The individual normalization/calibration amplitudes form a baseline image that is stored in the computer. As described above, the baseline image may also be acquired on a symmetric tissue region, such as the contralateral kidney or a symmetric tissue region of the same organ for internal tissue examination. The normalization process can be repeated several times to account for drifts in the individual elements. During the measurement process, the personal computer can adjust the gain of each AGC 3 4 based on the calibration values that account only for the electronic drift. Then, the defected image is subtracted from the baseline image of the examined tissue. ,Alternatively, while collecting the measurement data on the examined tissue, the measurement image is subtracted from the baseline image to create the tissue image that includes any tissue in homogeneities such as a tumor or bleeding. The sample-and-hold circuit maybe an analog circuit or the sample-and-hold ' function, including the filtering, may be performed digitally.

Fig. 8D shows diagramatically an amplitude cancellation imaging system employing a frequency multiplex method. Amplitude cancellation system 300 includes 21 oscillators 302 operating a frequencies in the range of 1 kHz to 100 kHz. Each oscillator 302 drives a light source 304 (for example, a laser diode or LED), which emits an intensity modulated light into the examined tissue. Each light detector 306 (for example, a photomultiplier, an avalanche photodiode PIN detector or a silicon detector) detects the intensity modulated light and provides a detector signal to an amplifier 308. The amplified detector signal is provided to a processing channel 310, which includes a band pass filter 312, an AGC 314, a lock-in amplifier 316, and a filter 318. Filter 312 filters the detector signal, and AGC 314 adjusts the amplitude according to the input signal from a personal computer. Lock-in amplifier 316 receives the amplified signal 315 and a reference signal 320 from oscillator 302. Lock-in amplifier 312 provides amplitude signal 317 to filter 318. Processing channel 310 may be an analog channel or a digital channel.

In the amplitude cancellation system 310, all light sources emit light at the same time into a selected tissue region. Each light source is modulated at a distinct frequency in the range of 1 kHz to 100 kHz. In order to resolve the modulated light signals and attribute them to the individual light sources, the oscillators operate at frequencies 1 kHz, 2 kHz, 4 kHz, 8 kHz, 16 kHz, ... Filters 312 and 318 are designed to provide only the detection signal from a selected light source, and lock-in amplifier 312 provides the amplitude of the signal at the selected frequency. Frequency multiplex system 300 is calibrated the same way as the time multiplex system 260, and the normalization/calibration amplitude values are also stored in the personal computer. The images are processed as described above.

All above-described optical systems will achieve a higher spacial resolution of the imaged tissue by increasing the number of sources and detectors. Furthermore, the sources and detectors may form various 1 dimensional, 1.5 dimensional, or 2 dimensional arrays as described in the above-referenced documents.

Before examination of a selected tissue region, the imaging system is first calibrated on a tissue model. The model data for different source-detector combinations is stored in a digital form. Alternatively, the model calibration may be performed by adjusting the detector gains prior to the tissue measurements. During the examination, the optical probe is placed over a designated body area, for example, a selected abdominal, thoracic, back or pelvic area of the body to target a selected organ. Two optical probes may be used to examine symmetrical organs: The images can be also acquired by taking advantage of *a priori* information obtained by X-ray tomography, an MRI or ultrasonic scan. The optical images are created using a back projection algorithm with or without correction for non-ballistic photon propagation (i.e., tissue absorption or scattering). The images may be displayed in the format of the tissue data minus the model data, or the right organ tissue data minus the left organ tissue data, for each wavelength (e.g., 750 and 830 nm).

The optical images may also be processed to image blood volume and blood oxygenation of the examined tissue. The blood volume image is the sum of 0.3 times the 750 nm data and 1.0 times the 830 nm data. The blood deoxygenation image is the difference of the 750 nm and the 830 nm data. The above coefficients, related to the absorption of oxy- and deoxy-hemoglobin, were derived from blood tests in model systems. The images have the highest specificity and sensitivity for symmetric organs or tissue regions, where the contralateral tissue region data is used as a baseline and both the blood volume data and the hemoglobin deoxygenation data is imaged and positionally compared.

Fig. 1C illustrates a pair of sources illuminating a pair of detectors with photon migration patterns intercepted by the head of a fetus. The distance between the sources and the detectors is 10 cm. The optical module is placed on the skin of the abdomen 350 in the pelvic area of woman 8 (Fig. 1). The location of the optical module may be determined by a prior ultrasound scan or by taking several optical images at varying locations. At the suitable position, most of the source - detector combinations generate banana patterns that penetrate the abdominal wall 352 and interine wall 354 and intercept different portions of the head 356 of the fetus 358. Some of the patterns are transmitted through the spaces not containing the head, which therefore provide a background signal. The background signal can be used to image the margin of the head.

Referring to Figs. 1A and 4, optical module 14 may be used with optical system 45. Optical module 14 has 9 sources and 4 detectors to be placed at distances of 9 cm apart on a 35 x 23 cm pad. (Optical modules of different sizes may be used at different stages of the pregnancy.) Imaging system 45 achieves phase cancellation in the detector, as described above. That is, two sources at the same wavelength, modulated with 0 phase and 180° phase, simultaneously illuminate the symmetrically located detector. Most of the banana-shaped optical patterns pass through the head and are thus appropriately perturbed by the absorption/scattering of the baby head. The imaging system uses a back projection algorithm to construct an image of the baby's - head with signals at both wavelengths. Thus, the processor can generate images of the blood volume and the blood deoxygenation of the examined region of the head of the fetus. These images may be used for routine examination of the fetus during, pregnancy. These images may also be used for long term monitoring of the fetus, where the optical module is worn by the pregnant woman. The above-described systems can not only image the head, or other parts of the fetus, they can also measure the blood oxygenation and non-invasively characterize the tissue of the fetus.

Alternatively, the above described imaging system may be used for monitoring during labor. After detecting the position of the fetal head, the optical module may be strapped in one location since the head is usually fixed in the cervix area. The imaging system would also provide the pulse rate of the fetus using the pulse oximetry technology. (See, for example, U.S. Patents 5,218,962; 4,869,254; 4,846,183; 4,700,708; 4,576,173 and the references cited therein) Based on the optical data (e.g., blood volume and oxygenation) and the detected pulse rate, the attending obstetrician can decide at any time whether to pursue vaginal delivery of the fetus or perform a C-section.

Prior to conducting the NIR transabdominal measurement, an ultrasound exam may be performed to determine the position of the fetal head, the placenta and the distance between the ultrasound transducer and the fetal brain. The optical probe is then placed on the maternal abdomen right above the pubic bone in a way that the sources and the detectors symmetrically straddled the location on the skin right above fetal brain. If the average distance between the surface of the fetal brain and the ultrasound transducer is about 2.5 cm, the optimal source and detector separation is about 10 cm in order to aim for a penetration depth of approximately 5 cm. To examine just the superficial maternal abdominal and uterine layers in a complementary measurement, the source and detector separation of 4 cm may be used. Prior to the measurement, a Doppler transducer and a pressure-sensitive monitor are attached to the maternal abdomen to monitor the fetal heart rate and uterine pressure, respectively. The optical apparatus calibration is performed when the fetal heart rate and uterine pressure are at a stable base line rate.

The optical measurement is synchronized with the fetal heart rate and uterine pressure using 760 nm and 850 nm wavelengths. The duration of the measurement may be approximately 30 minutes (duration of the antepartum NS). The optical density (O.D.) at each wavelength was calculated in order to account for the different base line calibration signals ($I_o$) at the two wavelengths for each patient. The incremented absorbance in $\Delta$O.D. was calculated using the following set of equations:

$$\Delta O.D. = \log\left(\frac{I_o}{I_{850nm}}\right) - \log\left(\frac{I_o}{I_{760nm}}\right)$$

$$\Sigma O.D. = 0.1 \, x \, \log\left(\frac{I_o}{I_{760nm}}\right) + \log\left(\frac{I_o}{I_{850nm}}\right)$$

where $\Delta$ O.D. is a measure of blood oxygenation, E O.D. is a measure of blood volume and $I_{760}$nm and $I_{850}$nm are the re-emitted signals at 760 and 850 nm, respectively.

To verify that the probe collects photons migrating trans-abdominally through the fetal head, the optical measurements are conducted in conjunction with vibro- acoustic stimulation of the fetus. Vibro- acoustic stimulation of the human fetus by means of artificial electronic larynx can reduce the false-positive and false-negative rates of NST. Vibro-acoustic stimulation is been used primarily to elicit accelerations in non-reactive fetal heart rates; this is considered a positive sign of fetal well being. Furthermore, the use of vibro acoustic simulation has been demonstrated to be a reliable means to achieve fetal heart rate activity.

A variety of tests and demonstrations have been put forward, each of which is consistent with the presence of a blood containing object within the uterus and in the position indicated by ultrasound. A quantitative validation the optical data is performed on the model of the near term maternal abdomen. Taking into account that the position of the model fetus should be matched with the *in vivo* fetus, ultrasound guidance permits matching of the fetal position in both systems. This principle of successive substitutions and better approximations of the model to the *in vivo* system affords a viable approach to instrumentation development and improvement.

The model uses a hemispherical spun copper mold into which a latex liner is poured in a thin uniform layer. This latex liner will simulate the sponge rubber pad in which the source and detectors are approximately located. An elastomer layer of low $\mu_a$ = 0.2 cm$^{-1}$ and $\mu_s$' = 10 cm$^{-1}$ is next poured into the model for thickness of 5 cm to simulate an adipose layer (a skin layer). A highly scattering material $\mu_a$ = 0.1, $\mu_s$' = 10 is poured into the model to simulate the musculature of the abdomen and of the uterus. This casting is held in place at the required thickness by an inner hemispherical shell. When solidified, the model is moved from the spun copper hemisphere and filled with water with small amounts of a scatterer (Intralipid) to simulate the turbid placental fluid.

The source/detector combinations are assembled on the outside of the model and observations are taken with and without a grapefruit sized object. A cellophane vessel with blood at 50 micromolar concentration which can be in the oxygenated or deoxygenated state. A number of studies can be carried out with the model head present and absent and filled with blood of various concentrations and oxygenation states, the latter being pumped through the model. The model consists of intralipid scattering factor $u_s$ = 10 cm$^{-1}$ $\mu_a$ = 0.01 cm$^{-1}$ filled with hemoglobin. This material is pumped through the model in the initial oxygenated state and upon the addition of yeast, in the deoxygenated state. Intermediate values of oxygenation are obtained without yeast by supplying the blood reservoir with oxygen/nitrogen values giving saturation between 5 and 95%. The following can be performed to optimize the system:

The concentration of blood can be varied from the standard hematocrit of 70 micromolar to 30 and 110 micromolar and the signal intensity for short and long pathlengths for the medial position of the simulated head are plotted. The oxygenation of hemoglobin at the three hematocrit are varied from 30% to 50% to 70% at the three blood concentrations mentioned above. Cross correlation plots of different blood concentration and blood oxygenation are made. Finally, yeast can be added to the blood model and transitions from the normoxic value of 70, 50, 30 and on to 0 are made in the medium position to test the validity of the arbitrary calibration of the probe.

The optical properties and thickness of the uterine tissue layer is varied and the changes in oxygenation of the fetal brain model can be evaluated for a fixed 50 $\mu$M concentration of hemoglobin. This can be used to determine the sensitivity and detection limits of the NIR signal to changes in oxygenation as a function of uterine optical properties and thickness as well.

The head can be placed 3 cm from the surface and translated parallel to and perpendicular to the long axes of the source detector combinations. The signal intensity is then plotted for short and long pathlengths. Furthermore, an imaging device can be designed based upon the modulation of the head position and the responses of the probes.

In addition to the rectangular probes shown in Figs. 1A and 1B, the above-described systems can use a concentric circle probe providing a wide variety of short and long paths, source-detector combinations afforded by the concentric circles of light sources and detectors. The optical probe with the concentric circles may use all light sources in a particular circle to illuminate the abdomen and the light detection can be localized in a single detector, or concentric circles of detectors. Imaging with at least three concentric circles of sources and detection can set up as the initial imaging system. When using the above-described amplitude cancellation and/or phase cancellation systems, the concentric circles probe defines the contours of fetal head and possibly the body as well.

As shown in Figs. 2, 2A and 2B, optical modules 12 or 14, located on the back of a subject, are used for in-vivo transabdominal or transthoracic examination of internal tissue. Any of the optical systems described in connection with Figs. 3 through 8D may be connected to one or several optical modules 12 or 14 to collect optical data from a tissue region of interest.

The optical system may generate single wavelength or multiple wavelength data sets of the examined tissue region, wherein the employed wavelength is sensitive to absorption or scattering by a tissue constituent (e.g., an endogenous or exogenous pigment, tissue cells, chemical compounds) or is sensitive to structural changes the examined tissue region. The optical data sets may represent tissue absorption, tissue scattering, or both. The optical data sets system may also generate blood volume and hemoglobin deoxygenation images, or images of any other tissue constituent, based on multiple wavelength optical data. A processor may use different image processing and enhancing algorithms known in the art. The processor may correlate several images to detect a suspicious tissue mass and to characterize the detected mass. The correlation includes determining congruency of the structures detected in different images. The processor may employ different types of combined scoring, based on several optical images alone or in combination with X-ray mammography, ultrasound examination, or fMRI, to characterize a suspicious tissue mass.

The blood volume and hemoglobin deoxygenation images provide an important tool for characterizing a suspicious anomaly in the examined tissue. While the blood volume and hemoglobin deoxygenation images, as well as the single wavelength images, are useful in locating an abnormal tissue region, these images are also used to characterize the metabolism or pathology of the suspicious tissue anomaly. Specifically, an increased blood volume signal is observed due to the increased vascularity of a tumor as a consequence of angiogenetic factors. These factors include actively metabolizing regions and necrotic/apoptotic regions of the tumor. On the other hand, the hemoglobin deoxygenation signal is related to metabolic intensity. That is, the balance between oxygen delivery and oxygen uptake, which in tumors is usually balanced in favor of oxygen uptake exceeding oxygen delivery. The increased oxygen uptake occurs particularly for those tumors that are aggressively growing, and may as well be metastatic.

By selecting an appropriate wavelength, or several wavelengths, sensitive to an optically active tissue property, the imaging system can non-invasively characterize a tissue anomaly. The above-mentioned wavelengths are sensitive to hemoglobin and hemoglobin oxygenation, but other wavelengths sensitive to absorption by any tissue constituent may be used. Furthermore, an optical contrast agent (e.g., cardiogreen, indocianine green) may be injected intravenously. The imaging system will then use a wavelength sensitive to the administered contrast agent. The regions of increased blood volume will also have a higher content of the contrast agent.

Alternatively, differences in tissue scattering may be imaged. Due to differences in the optical refractive index, different types of tissue and different tissue solutes scatter light differently. The above-described imaging systems are also sensitive to scattering changes. The imaging system may use a wavelength that does not exhibit absorption changes for different types of tissue or different tissue solutes, but exhibits differences in scattering.

Non-invasive characterization of tissue may be performed by combining the data from the above described images. For example, a two dimensional data chart may display blood volume (i.e., vasculogenesis) vs. blood deoxygenation (i.e. hypermetabolism) for a "suspicious" tissue region using the model data as a reference, using a symmetrical tissue region as a reference, or using a symmetrical organ data as a reference.

The imaging system performs the following tissue characterization by co-registration of several images. In principle, vasculogenesis (blood volume) and hypermetabolism (tissue hypoxia) occur in similar and often identical tissue volumes. Thus, the two images would show pronounced structures in the same location. The vascular volume is represented by the blood volume signal. The imaging systems evaluates the congruence of the two image structures in order to locate a suspicious tissue region. The first step is the normalization of the two images to equalize the maximum signals. A computer program selects the area and obtains the integrated value for the spatial congruence residual and for the blood volume signal. Then, subtraction pixel-by-pixel gives an image that provides a residual value used to estimate the congruence of the two shapes obtained from the blood volume and deoxygenation images. A simpler procedure is to take the maximum value of the difference and divide it by the maximum value of the normalized value for the two images.

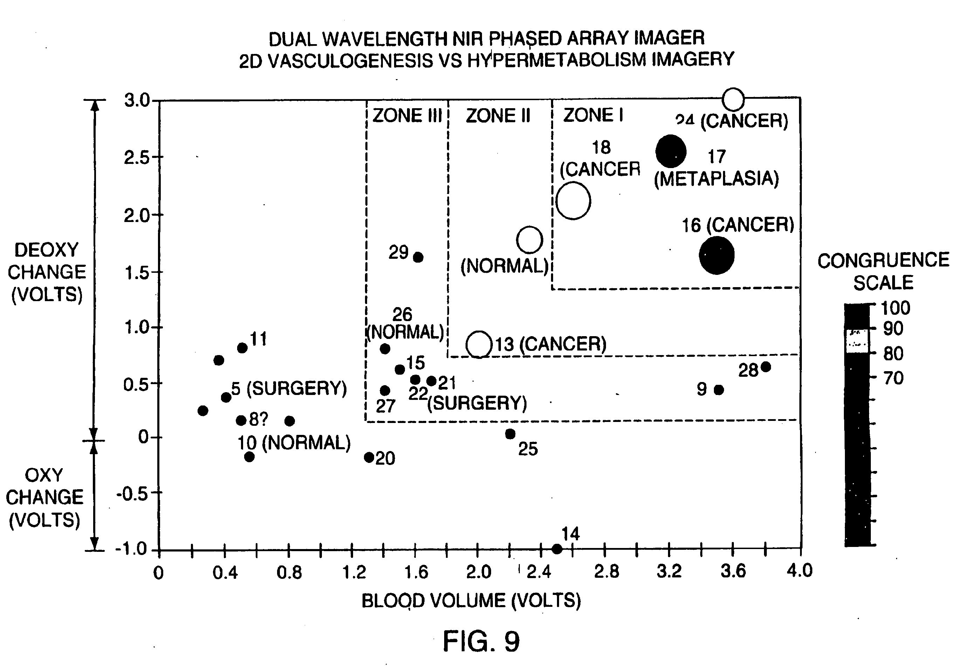

Referring to Fig. 9, a "four" dimensional graph may be used to summarize images of suspicious regions (Here Fig. 9 is only a proposed technique for data evaluation and does not show actual tissue data). The blood volume (measured in volts) is plotted on the abscissa and deoxygenation (measured in volts) is plotted on the ordinate. The measured size of a suspicious mass is depicted as a circle diameter and the percentage congruence between the blood volume image and the deoxygenation image of the suspicious mass can be shown using a color scale. The percentage of congruence signals may be given in a color scale based on the following formula:

$$1 - \left( \frac{maximum\ overlap\ residual}{maximum\ blood\ volume\ signal} \right) \times 100$$

The "four" dimensional diagram is based on the following:

1. The size of the image of a suspicious mass (plotted as one half its longest dimension).
2. The congruence of blood volumes and blood deoxygenation in color.
3. The blood volume in the congruent region measured in volts (scale of the abscissa).
4. Blood deoxygenation in the congruent region in volts (scale of the ordinate).

The four-dimensional nature of Fig. 9 permits the assignment of sensitivity and specificity according to the signal strength of blood volume data and the signal strength of deoxygenation data. I divided the region of signals in Fig. 9 into four zones. Zone I was defined for blood volume values above about 2.4 V and deoxygenation values above about 1.4 V. Zone II, located below Zone I, was defined for blood volume values above about 1.7 V and deoxygenation values above about 0.75 V. Zone III, located below Zone II, was defined for blood volume values above about 1.3 V and deoxygenation values above about 0.2 V. Zone IV was located below Zone III. Zones III and II may likely include cancerous masses which are expected to provide high blood volume and deoxygenation signals.

The image structures to be evaluated in the optical images may be selected using X-ray, ultrasound or MRI data. Alternatively, image structures may be based upon "suspicious mass" guidance only, using the contralateral tissue data as a reference, or using the model data as a reference. The use of the contralateral tissue (i.e., the symmetric tissue) reduces the signals from abnormal tissue (e.g., non-cancerous tissue), but the measurement on the symmetric tissue is not always feasible.

Additional embodiments are within the following claims.

**Claims**

1. An optical method for *in vivo,* non-invasive, transabdominal examination of fetal tissue comprising:

   providing an optical module (12, 14, 212) including an array of optical input ports optically coupled to light sources ($S_1$, $S_2$, $S_3$, ...) and detection ports optically coupled to light detectors ($D_1$, $D_2$, $D_3$, ...); located in a selected geometrical pattern that provide a multiplicity of photon migration paths inside the uterus of a pregnant female subject;
   placing said optical module on the exterior of the abdomen of the pregnant female subject;
   introducing visible or infrared light from at least one said optical input port into the uterus and receiving photons that have migrated in the uterus to at least one of said detection ports;
   detecting said received photons by at least one optical detector optically coupled to said least one detection port;
   controlling said introducing and detecting steps to collect optical data corresponding to photons of light that have partially migrated inside a fetal tissue region;
   forming at least two data sets, a first of said data sets representing blood volume in the fetal tissue region and a second of said data sets representing blood oxygenation the fetal tissue region; and
   processing said optical data by correlating said first and second data sets to characterize the fetal tissue region.

2. The optical method of claim 1 wherein said processing includes determining hemoglobin oxygenation of said fetal tissue.

3. The optical method of claim 1 further including calibrating said optical module.

4. The optical method of claim 1 further including stimulating said fetal tissue.

5. The optical method of claim 1 wherein said processing includes monitoring selected tissue region of said fetal tissue.

6. The optical method of claim 5 wherein said monitoring includes monitoring during delivery.

7. The optical method of claim 1 wherein said processing includes imaging selected tissue region of said fetal tissue.

8. The optical method of claim 1 or 2 wherein said processing includes correlating said first and second data sets by determining congruence between data of said two sets.

9. The optical method of claim 8 wherein said processing includes ordering said first and second data sets as two-dimensional images and to determine said congruence using said two-dimensional images.

10. The optical method of claim 8 wherein said processing includes ordering said first and second data sets as two-dimensional images and to determine said congruence using the following formula:

$$1 - \left( \frac{maximum \; overlap \; residual}{maximum \; selected \; tissue \; signal} \right) x100$$

11. The optical method of claim 1 wherein said processing includes determining a pulse rate of the fetus.

12. The optical method of claim 1 wherein said controlling step includes collecting said optical data corresponding to photons that have partially migrated inside brain tissue of the fetus.

13. The optical method of claim 1 wherein said placing step includes moving said optical module on the exterior of the abdomen to relocate said photon migration paths inside the uterus so that said optical data correspond to photons that have partially migrated inside brain tissue of the fetus.

14. The optical method of claim 1 further including locating the head of the fetus by using an ultrasound system and then, based on said locating, performing said placing of said optical module on the exterior of the abdomen to position said photon migration paths inside the uterus so that said optical data correspond to photons that have partially migrated inside brain tissue of the fetus.

15. The optical method of claim 12, 13 or 14 wherein said processing includes determining hemoglobin oxygenation of said brain tissue.

16. The optical method of claim 12, 13 or 14 wherein said processing includes determining a pulse rate of the fetus.

17. The optical method of claim 12, 13 or 14 wherein said processing includes evaluating said brain tissue.

18. The optical method of claim 12, 13 or 14 wherein said processing includes creating an image of said brain tissue.

19. The optical method of claim 18 wherein said processing includes creating images of blood volume in said brain tissue and blood oxygenation in said brain tissue.

20. The optical method of claim 19 wherein said images include hemoglobin oxygenation data.

21. The optical method of claim 19 wherein said images include hemoglobin deoxygenation data.

22. The optical method of claim 18 wherein said image includes data sensitive to a tissue constituent.

23. The optical method of claim 12.13 or 14 wherein said processing includes forming at least two data sets, a first of said data sets representing blood volume in said brain tissue and a second of said data sets representing blood oxygenation said brain tissue; and the method further including correlating said first and second data sets to detect abnormal tissue in said brain tissue.

24. The optical method of claim 1, 3, 4 or 5 wherein said light source emits light of a wavelength sensitive to an endogenous pigment.

25. The optical method of claim 24 wherein said endogenous pigment is hemoglobin.

26. The optical methods of claim 1, 3, 4 or 5 wherein said light source emits light of a wavelength sensitive to an exogenous pigment.

**27.** The optical method of claim 26 in which said exogenous pigment is a selected contrast agent.

**28.** The optical method of claim 1 or 2 further including generating a first carrier waveform at a first frequency on the order of magnitude of $10^8$Hz, said first frequency having a time characteristic compatible with the time delay of photon migration between said input and said detection ports, the method further including:

generating said light modulated by said first carrier waveform;
determining a change in the waveform of the detected light relative to the waveform of the introduced light and determining therefrom the phase shift of said detected light at said wavelength, said phase-shifted light being indicative of scattering or absorptive properties of the examined tissue region; and
forming said data sets based on the measured phase shift.

**29.** The optical method of claim 28 further including generating a second waveform at a second frequency; the method further including

providing to said detector a reference waveform at a reference frequency offset by a frequency on the order of magnitude of $10^3$Hz from said first frequency,
producing a signal, at said offset frequency, corresponding to light of said detected photons; and
comparing, at said offset frequency, the detected light with the introduced light and to determine therefrom said phase shift.

**30.** The optical method of claim 1 or 2 further including generating a first carrier waveform of a selected frequency compatible with time delay of photon migration from said input port to said detection port, providing a phase splitter connected to receive said carrier waveform, and providing first and second double balanced mixers; the method further including:

providing said carrier waveform to one of said light sources and generate light modulated at said frequency;
producing by said phase splitter first and second reference phase signals of predefined substantially different phases;
producing an in-phase output signal and a quadrature output signal by said first and second double balanced mixers receiving said first and second reference phase signals, respectively, and
forming said data sets using said in-phase output signal and said quadrature output signal.

**31.** The optical method of claim 30 further including calculating a phase shift ($\Theta_\lambda$) between said introduced light and said detected light to forming said data set.

**32.** The optical method of claim 30 further including calculating an average migration pathlength of photons scattered in the examined tissue between said optical input and detection ports prior to forming said data set.

**33.** The optical method of claim 32 further including quantifying hemoglobin saturation (Y) using said pathlength.

**34.** The optical method of claim 30 further including calculating a signal amplitude ($A_\lambda$) determined as a square root of a sum of squares of said in-phase output signal and said quadrature output signal prior to forming said data set.

**35.** The optical method of claim 1 or 2 further including at least one oscillator constructed to generate a carrier waveform of a selected frequency, a controller constructed to control the emitted light intensity or phase relationship of patterns simultaneously introduced from multiple input ports, said introduced patterns forming resulting light that possesses a substantial gradient of photon density in at least one direction, said resulting light being scattered and absorbed over said migration paths; the method further including:

generate said light being intensity modulated at said frequency to achieve a known light pattern;
detecting over time the resulting light that has migrated in the tissue, and
processing signals of said detected resulting light in relation to said introduced light to create said data sets indicative of influence of the examined tissue upon said substantial gradient of photon density of said resulting light.

**36.** The optical method of claim 35 further including detecting a phase of the detected light.

**37.** The optical method of claim 35 further including detecting an amplitude of the detected light.

**38.** The optical method of claim 35 wherein the phase relationship of light patterns introduced from two input ports is 180 degrees.

**39.** The optical method of claim 1 or 2 wherein said light sources produce relatively long light pulses and said processing including subtracting amplitude of two said pulses emitted from two input ports located symmetrically relative to one detection port.

**40.** An optical system (15, 45, 100, 150, 200, 260, 300) for *in vivo,* non-invasive, transabdominal examination of fetal tissue comprising:

an optical module (12, 14, 212) including an array of optical input ports and detection ports located in a selected geometrical pattern to provide a multiplicity of photon migration paths inside the uterus, each said optical input port being constructed to introduce visible or infrared light emitted from a light source ($S_1$, $S_2$, $S_3$, ...), each said optical detection port being constructed to receive photons of light that have migrated from at least one of said input ports and provide said received light to a light detector ($D_1$, $D_2$, $D_3$, ...);
a controller (19, 49, 140, 175, 262, 264) constructed and arranged to control operation of said light source and said light detector to detect photons that have migrated over at least one of said photon migration paths inside fetal tissue; and
a processor (PC) connected to receive signals from said detector, said processor being co-operatively arranged with said controller to control operation of said light source and said light detector to detect photons that have migrated over photon migration path including said fetal tissue, said processor being also arranged to form at least two data sets, a first of said data sets representing blood volume in the fetal tissue region and a second of said data sets representing blood oxygenation in the fetal tissue region; said processor being arranged to correlate said first and second data sets to characterize the examined fetal tissue region.

**41.** The optical system of claim 40 wherein said controller and said processor are arranged to evaluate said optical data and subsequently control operation of said light source and said light detector to collect additional optical data corresponding to photons that have partially migrated inside brain tissue of the fetus.

**42.** The optical system of claim 40 or 41 wherein said processor is further arranged to determine a pulse rate of the fetus.

**43.** The optical system of claim 40, 41 or 42 wherein said optical module, including said light source and said light detector, is constructed to be positioned in contact with the abdomen.

**44.** The optical system of claim 43 further including a barrier material located between said optical module, including said light source and said light detector, and the skin.

**45.** The optical system of claim 40, 41 or 42 wherein said optical module includes a multiplicity of optical fibers providing optical coupling between said light source and said light detector and the abdomen.

**46.** The optical system of claim 40, 41 or 42 wherein said optical module is constructed to include said pattern having each said light source surrounded by several said light detectors.

**47.** The optical system of claim 46 wherein said light detectors provide at least two symmetrical paths for each said light source.

**48.** The optical system of claim 40, 41 or 42 wherein said optical module is constructed to include said pattern having each said light detector surrounded by several said light sources.

**49.** The optical system of claim 48 wherein said light sources provide at least two symmetrical paths for each said light detector.

**50.** The optical system of claim 40, 41, 42, 43, 45 or 46 wherein said processor is further arranged to determine hemoglobin oxygenation of said fetal tissue.

**51.** The optical system of claim 40, 41, 42, 43, 45 or 46 wherein said optical module is constructed for placement on the abdomen based on locating the head of the fetus by an ultrasound system so that said optical data correspond to photons that have partially migrated inside brain tissue of the fetus.

52. The optical system of claim 41 wherein said processor is arranged to determine hemoglobin oxygenation of said brain tissue.

53. The optical apparatus of claim 41 wherein said processor is arranged to create an image said brain tissue.

54. The optical system of claim 41 wherein said processor is arranged to create images blood volume in said brain tissue and blood oxygenation in said brain tissue.

55. The optical system of claim 40, 41, 42, 43, 45 or 46 wherein said processor is arranged to correlate said first and second data sets by determining congruence between data of said two sets.

56. The optical system of claim 40 wherein said processor is programmed to order said first and second data sets as two-dimensional images and to determine said congruence using said two-dimensional images.

57. The optical system of claim 40 wherein said processor is programmed to order said first and second data sets as two-dimensional images and to determine said congruence using the following formula:

$$1 - \left( \frac{maximum\ \ overlap\ \ residual}{maximum\ \ selected\ \ tissue\ \ signal} \right) x100$$

58. The optical system of claim 40, 41 or 42 wherein said processor is adapted to produce from said data set an image data set by implementing an optical tomography algorithm.

59. The optical system of claim 58 in which said optical tomography algorithm employs factors related to determined probability distribution of photons attributable to the scattering character of the tissue being imaged.

60. The optical system of claim 40, 41, 42, 43, 45 or 46 further including a display device constructed to receive said image data set from said processor and to display an image.

61. The optical system of claim 40, 41, 42, 43, 45 or 46 constructed to introduce and detect photons at two wavelengths selected to provide sensitivity to a tissue constituent.

62. The optical system of claim 40, 41, 42, 43, 45 or 46 wherein said light source emits light of a wavelength sensitive to an endogenous pigment.

63. The optical system of claim 62 wherein said endogenous pigment is hemoglobin.

64. The optical system of claim 40, 41, 42, 43, 45 or 46 wherein said light source emits light of a wavelength sensitive to an exogenous pigment.

65. The optical system of claim 64 in which said exogenous pigment is a selected contrast agent.

66. The optical system of claim 40, 41 or 42 further comprising

a first oscillator constructed to generate a first carrier waveform at a first frequency on the order of magnitude of $10^8$Hz, said first frequency having a time characteristic compatible with the time delay of photon migration between said input and detection ports;
said light source being coupled to said first oscillator and constructed to generate said light modulated by said first carrier waveform;
a phase detector constructed to determine change in waveform of the detected light relative to the waveform of the introduced light and measure therefrom the phrase shift of said detected light at said wavelength, said phase-shifted light being indicative of scattering or absorptive properties of the examined tissue region; and

said processor being arranged to form said data sets based on the measured phase shift.

**67.** The optical system of claim 66 further comprising

a second oscillator constructed to generate a second waveform at a second frequency;
said detector being arranged to receive a reference waveform at a reference frequency offset by a frequency on the order of magnitude of $10^3$Hz from said first frequency and to produce a signal, at said offset frequency, corresponding to said detected radiation; and
said phase detector being adapted to compare, at said offset frequency, the detected light with the introduced light and to determine therefrom said phase shift.

**68.** The optical system of claim 40, 41 or 42 further comprising:

an oscillator constructed to generate a first carrier waveform of a selected frequency compatible with time delay of photon migration from said input port to said detection port; said light source being connected to receive from said oscillator said carrier waveform and constructed to generate optical light modulated at said frequency;
a phase splitter connected to receive said carrier waveform from said oscillator and produce first and second reference phase signals of predefined substantially different phases;
first and second double balanced mixers connected to receive from said phase splitter said first and second reference phase signals, respectively, and connected to receive from said detector said detector signal and to produce therefrom an in-phase output signal and a quadrature output signal, respectively; and
said processor being connected to said double balanced mixers and arranged to receive said in-phase output signal and said quadrature output signal and form therefrom said data sets.

**69.** The optical system of claim 68 wherein said processor is arranged to calculate a phase shift ($\Theta_\lambda$) between said light introduced at said input port and said light detected at said detection port prior to forming said data set.

**70.** The optical system of claim 68 wherein said processor is arranged to calculate an average migration pathlength of photons scattered in the examined tissue between said optical input port and said optical detection port prior to forming said data set.

**71.** The optical system of claim 70 wherein said processor further employs said pathlength in quantifying hemoglobin saturation (Y) of the examined tissue.

**72.** The optical system of claim 68 wherein said processor is arranged to calculate a signal amplitude ($A_\lambda$) determined as a square root of a sum of squares of said in-phase output signal and said quadrature output signal prior to forming said data set.

**73.** The optical system of claim 72 further comprising:

a narrow band detector connected to receive from said optical detector said detector signal and to produce a DC output signal therefrom; and
said signal processor further determining a modulation index ($M_\lambda$) as a ratio of values of said signal amplitude and said signal amplitude plus said DC output signal.

**74.** The optical system of claim 40, 41 or 42 further comprising:

at least one oscillator constructed to generate a carrier waveform of a selected frequency, said light source being operatively connected to said oscillator constructed to generate light of a visible or infrared wavelength, said light being intensity modulated at said frequency to achieve a known light pattern;
said controller constructed to control the emitted light intensity or phase relationship of patterns simultaneously introduced from multiple input ports, said introduced patterns forming resulting light that possesses a substantial gradient of photon density in at least one direction, said resulting light being scattered and absorbed over said migration paths;
said detector constructed and arranged to detect over time the resulting light that has migrated in the tissue to said detection port, and
said processor being further arranged to process signals of said detected resulting light in relation to said introduced light to create said data sets indicative of influence of the examined tissue upon said substantial gradient of photon density of said resulting radiation.

75. The optical system of claim 74 further comprising a phase detector constructed to detect the phase of the detected light and provide said phase to said processor.

76. The optical system of claim 74 further comprising an amplitude detector constructed to detect the amplitude of the detected light and provide said amplitude to said processor.

77. The optical system of claim 74 wherein the phase relationship of light patterns introduced from two input ports is 180 degrees.

78. The optical system of claim 40, 41 or 42 wherein said light source produces relatively long light pulses and the processor forms said data sets by subtracting amplitude of two said pulses emitted from two input ports located symmetrically relative to one detection port.

**Patentansprüche**

1. Optisches Verfahren zur nicht-invasiven, transabdominalen in-vivo Untersuchung von fetalem Gewebe, welches Folgendes aufweist:

   Vorsehen eines optischen Moduls (12, 14, 212), welches eine Anordnung von optischen Eingangsanschlüssen, die optisch mit Lichtquellen ($S_1$, $S_2$, $S_3$, ...) gekoppelt sind, und Detektionsanschlüssen, die optisch mit Lichtdetektoren ($D_1$, $D_2$, $D_3$, ...) gekoppelt sind, aufweist, welche in einem ausgewählten geometrischen Muster angeordnet sind, welche eine Vielzahl von Photonen-Migrationspfaden innerhalb des Uterus bzw. der Gebärmutter einer schwangeren Frau vorsehen;
   Platzieren des optischen Moduls auf die Außenseite des Abdomens der schwangeren Frau;
   Einführen von sichtbarem oder infrarotem Licht von mindestens einem optischen Eingangsanschluss in die Gebärmutter und Empfangen von Photonen, die in der Gebärmutter zu mindestens einem der Detektionsanschlüsse migriert bzw. gewandert sind;
   Detektieren der empfangenen Photonen durch mindestens einen optischen Detektor, der optisch mit mindestens einem Detektionsanschluss gekoppelt ist;
   Steuern der Einführ- und Detektionsschritte, um optische Daten zu sammeln, die den Lichtphotonen entsprechen, die teilweise innerhalb einer fetalen Geweberegion gewandert sind;
   Bilden von mindestens zwei Datensätzen, wobei ein erster der Datensätze das Blutvolumen in der fetalen Geweberegion repräsentiert und wobei ein zweiter der Datensätze die Blutoxygenierung der fetalen Geweberegion repräsentiert; und
   Verarbeiten der optischen Daten durch Korrelieren der ersten und zweiten Datensätze, um die fetale Geweberegion zu charakterisieren.

2. Optisches Verfahren nach Anspruch 1, wobei die Verarbeitung das Bestimmen der Hämoglobinoxygenierung des fetalen Gewebes beinhaltet.

3. Optisches Verfahren nach Anspruch 1, welches ferner das Kalibrieren des optischen Moduls beinhaltet.

4. Optisches Verfahren nach Anspruch 1, welches ferner das Stimulieren des fetalen Gewebes beinhaltet.

5. Optisches Verfahren nach Anspruch 1, wobei das Verarbeiten das Überwachen einer ausgewählten Geweberegion des fetalen Gewebes beinhaltet.

6. Optisches Verfahren nach Anspruch 1, wobei das Überwachen die Überwachung während der Geburt beinhaltet.

7. Optisches Verfahren nach Anspruch 1, wobei das Verarbeiten das Abbilden einer ausgewählten Geweberegion des fetalen Gewebes beinhaltet.

8. Optisches Verfahren nach Anspruch 1 oder 2, wobei das Verarbeiten das Korrelieren der ersten und zweiten Datensätze durch Bestimmen der Kongruenz zwischen den zwei Datensätzen beinhaltet.

**9.** Optisches Verfahren nach Anspruch 8, wobei das Verarbeiten das Anordnen der ersten und zweiten Datensätze als zweidimensionale Abbildungen und das Bestimmen der Kongruenz unter Verwendung der zweidimensionalen Abbildungen beinhaltet.

**10.** Optisches Verfahren nach Anspruch 8, wobei das Verarbeiten das Anordnen der ersten und zweiten Datensätze als zweidimensionale Abbildungen und das Bestimmen der Kongruenz unter Verwendung der folgenden Formel beinhaltet:

$$1 - \left( \frac{\text{maximale Überlappungsrestgröße}}{\text{maximales Signal des ausgewählten Gewebes}} \right) x\ 100$$

**11.** Optisches Verfahren nach Anspruch 1, wobei das Verarbeiten das Bestimmen einer Pulsfrequenz des Feten beinhaltet.

**12.** Optisches Verfahren nach Anspruch 1, wobei der Steuerungsschritt das Sammeln optischer Daten beinhaltet, die den Photonen entsprechen, welche teilweise innerhalb des Hirngewebes des Feten gewandert sind.

**13.** Optisches Verfahren nach Anspruch 1, wobei der Platzierungsschritt das Bewegen des optischen Moduls auf der Außenseite des Abdomens beinhaltet, um die Photonenmigrationspfade innerhalb der Gebärmutter so zu versetzen, dass die optischen Daten den Photonen entsprechen, die teilweise innerhalb des Hirngewebes des Feten beinhaltet bzw. gewandert sind.

**14.** Optisches Verfahren nach Anspruch 1, welches ferner beinhaltet, den Kopf des Feten unter Verwendung eines Ultraschallsystems zu lokalisieren bzw. zu orten und dann, auf der Grundlage der Ortung, das Platzieren des optischen Moduls auf der Außenseite des Abdomens durchzuführen, um die Photonenmigrationspfade innerhalb der Gebärmutter so zu positionieren, dass die optischen Daten den Photonen entsprechen, die teilweise innerhalb des Hirngewebes des Feten gewandert sind.

**15.** Optisches Verfahren nach Anspruch 12, 13 oder 14, wobei das Verarbeiten aufweist, die Hämoglobinoxygenierung des Hirngewebes zu bestimmen.

**16.** Optisches Verfahren nach Anspruch 12, 13 oder 14, wobei das Verarbeiten aufweist, eine Pulsfrequenz des Feten zu bestimmen.

**17.** Optisches Verfahren nach Anspruch 12, 13 oder 14, wobei das Verarbeiten aufweist, das Hirngewebe zu bewerten bzw. zu evaluieren.

**18.** Optisches Verfahren nach Anspruch 12, 13 oder 14, wobei das Verarbeiten aufweist, eine Abbildung des Hirngewebes zu erzeugen.

**19.** Optisches Verfahren nach Anspruch 18, wobei das Verarbeiten aufweist, Abbildungen des Blutvolumens in dem Hirngewebe und die Blutoxygenierung in dem Hirngewebe zu erzeugen.

**20.** Optisches Verfahren nach Anspruch 19, wobei die Abbildungen Hämoglobinoxygenierungsdaten aufweisen.

**21.** Optisches Verfahren nach Anspruch 19, wobei die Abbildungen Hämoglobindeoxygenierungsdaten aufweisen.

**22.** Optisches Verfahren nach Anspruch 18, wobei die Abbildung Daten aufweist, die auf einen Gewebsbestandteil empfindlich sind.

**23.** Optisches Verfahren nach Anspruch 12, 13 oder 14, wobei die Verarbeitung beinhaltet, mindestens zwei Datensätze zu bilden, wobei ein erster der Datensätze, das Blutvolumen in dem Hirngewebe repräsentiert und eine zweiter der Datensätze die Blutoxygenierung des Hirngewebes repräsentiert, und wobei das Verfahren ferner das Korrelieren der ersten und zweiten Datensätze aufweist, um abnormes Gewebe in dem Hirngewebe zu erkennen bzw. zu detektieren.

24. Optisches Verfahren nach Anspruch 1, 3, 4 oder 5, wobei die Lichtquelle Licht einer Wellenlänge emittiert, die auf ein endogenes Pigment empfindlich ist.

25. Optisches Verfahren nach Anspruch 24, wobei das endogene Pigment Hämoglobin ist.

26. Optisches Verfahren nach Anspruch 1, 3, 4 oder 5, wobei die Lichtquelle Licht einer Wellenlänge emittiert, die auf ein exogenes Pigment empfindlich ist.

27. Optisches Verfahren nach Anspruch 26, in dem das exogene Pigment ein ausgewähltes Kontrastmittel ist.

28. Optisches Verfahren nach Anspruch 1 oder 2, welches ferner aufweist, eine erste Trägerwellenform mit einer ersten Frequenz in der Größenordnung von $10^8$ Hz zu erzeugen, wobei die erste Frequenz eine Zeitcharakteristik besitzt, die zu der Zeitverzögerung der Photonenmigration zwischen den Eingangs- und Detektionsanschlüssen kompatibel ist, wobei das Verfahren ferner Folgendes aufweist:

Erzeugen des Lichtes, das durch die erste Trägerwellenform moduliert ist;
Bestimmen einer Veränderung der Wellenform des detektierten Lichtes relativ zu der Wellenform des eingeführten Lichtes und daraus Bestimmen der Phasenverschiebung des detektierten Lichtes bei dieser Wellenlänge, wobei das phasenverschobene Licht eine Anzeige bildet für die Streuungs- oder Absorptionseigenschaften der untersuchten Geweberegion; und
Bilden der Datensätze auf der Grundlage der gemessenen Phasenverschiebung.

29. Optisches Verfahren nach Anspruch 28, welches ferner aufweist, eine zweite Wellenform mit einer zweiten Frequenz zu erzeugen, wobei das Verfahren ferner Folgendes aufweist:

Liefern einer Referenzwellenform an den Detektor mit einer Referenzfrequenz, die um eine Frequenz in der Größenordnung von $10^3$ Hz zur ersten Frequenz versetzt ist, Produzieren eines Signals mit der Ausgleichs- bzw. Offset-Frequenz, die dem Licht der detektierten Photonen entspricht; und
Bei der Offsets-Frequenz Vergleichen des detektierten Lichtes mit dem eingeführten Licht und bestimmen der Phasenverschiebung daraus.

30. Optisches Verfahren nach Anspruch 1 oder 2, welches ferner aufweist, eine erste Trägerwellenform mit einer ausgewählten Frequenz zu erzeugen, die mit der Zeitverzögerung der Photonenmigration von dem Eingangsanschluss zu dem Detektionsanschluss kompatibel ist, Vorsehen eines Phasensplitters bzw. Phasenteilers, der angeschlossen ist, um die Trägerwellenform zu empfangen, und Vorsehen von ersten und zweiten Doppelgegentaktmischern (double balanced mixers), wobei das Verfahren ferner Folgendes aufweist:

Liefern der Trägerwellenform an eine der Lichtquellen und erzeugen von Licht, das mit dieser Frequenz moduliert ist;
Erzeugen von ersten und zweiten Phasenreferenzsignalen von vorbestimmten, im Wesentlichen unterschiedlichen Phasen durch den Phasensplitter;
Erzeugen eines In-Phase-Ausgangssignals und eines Quadratur-Ausgangssignals durch die ersten und zweiten Doppelgegentaktmischer, welche die ersten bzw. zweiten Phasenreferenzsignale empfangen, und
Bilden von Datensätzen, die das In-Phase-Ausgangssignal und das Quadratur-Ausgangssignal verwenden.

31. Optisches Verfahren nach Anspruch 30, welches ferner das Berechnen einer Phasenverschiebung ($\theta_\lambda$) zwischen dem eingeführten Licht und dem detektierten Licht aufweist, um den Datensatz zu bilden.

32. Optisches Verfahren nach Anspruch 30, welches ferner das Berechnen einer durchschnittlichen Migrationspfadlänge der Photonen aufweist, die in dem untersuchten Gewebe zwischen den optischen Eingangs- und den Detektionsanschlüssen gestreut sind, bevor der Datensatz gebildet wird.

33. Optisches Verfahren nach Anspruch 32, welches ferner das Quantifizieren der Hämoglobinsättigung (Y) aufweist, unter Verwendung der Pfadlänge.

**34.** Optisches Verfahren nach Anspruch 30, welches ferner das Berechnen einer Signalamplitude ($A_\lambda$) aufweist, die bestimmt ist als Quadratwurzel einer Summe von dem Quadrat des In-Phase-Ausgangssignals und dem Quadrat des Quadratur-Ausgangssignals, und zwar vor Bildung des Datensatzes.

**35.** Optisches Verfahren nach Anspruch 1 oder 2, welches ferner Folgendes aufweist: mindestens einen Oszillator, der konstruiert ist, um eine Trägerwellenform mit ausgewählter Frequenz zu erzeugen, eine Steuervorrichtung, die konstruiert ist, um die Intensität des emittierten Lichtes oder das Phasenverhältnis von Mustern zu steuern, die gleichzeitig von mehreren Eingangsanschlüssen eingeführt werden, wobei die eingeführten Muster ein resultierendes Licht bilden, welches einen wesentlichen Gradienten der Photonendichte in mindestens eine Richtung besitzt, wobei das resultierende Licht über die Migrationspfade gestreut und absorbiert wird; wobei das Verfahren weiter Folgendes aufweist:

Erzeugen eines Lichtes, welches bei der Frequenz in der Intensität moduliert ist, um ein bekanntes Lichtmuster zu erhalten;
Detektieren bezüglich der Zeit von resultierenden Licht, das in dem Gewebe gewandert ist, und
Verarbeiten der Signale des detektierten resultierenden Lichtes in Beziehung zu dem eingeführten Licht, um die Datensätze zu erzeugen, die eine Anzeige bilden für den Einfluss des untersuchten Gewebes auf den substantiellen Gradienten der Photonendichte des resultierenden Lichts.

**36.** Optisches Verfahren nach Anspruch 35, welches das Detektieren einer Phase des detektierten Lichtes aufweist.

**37.** Optisches Verfahren nach Anspruch 35, welches ferner das Detektieren einer Amplitude des detektierten Lichtes aufweist.

**38.** Optisches Verfahren nach Anspruch 35. bei dem die Phasenbeziehung der Lichtmuster, die von zwei Eingangsanschlüssen eingeführt werden, 180° beträgt.

**39.** Optisches Verfahren nach Anspruch 1 oder 2, wobei die Lichtquellen relativ lange Lichtimpulse erzeugen und das Verarbeiten das Subtrahieren der Amplitude von zwei dieser Impulse aufweist, die von zwei Eingangsanschlüssen emittiert werden, welche relativ zu einem Detektionsanschluss symmetrisch gelegen sind.

**40.** Optisches System (15, 45, 100, 150, 200, 260, 300) zur nicht-invasiven, transabdominalen In-vivo-Untersuchung von fetalem Gewebe, welches Folgendes aufweist:

ein optisches Modul (12, 14, 212), welches eine Anordnung von optischen Eingangsanschlüssen und Detektionsanschlüssen aufweist, die in einem ausgewählten geometrischen Muster angeordnet sind, um eine Vielzahl von Photonen-Migrationspfaden innerhalb der Gebärmutter bzw. des Uterus vorzusehen, wobei jeder optische Eingangsanschluss konstruiert ist, um sichtbares oder infrarotes Licht einzuführen, welches von einer Lichtquelle ($S_1$, $S_2$, $S_3$, ...) emittiert wird, wobei jeder optische Detektionsanschluss konstruiert ist, um Lichtphotonen zu empfangen, die von mindestens einem der Eingangsanschlüsse gewandert sind und das empfangene Licht zu einem Lichtdetektor ($D_1$, $D_2$, $D_3$, ...) liefern;
eine Steuervorrichtung (19, 49, 140, 175, 262, 264), die konstruiert und angeordnet ist, um den Betrieb der Lichtquelle und der Lichtdetektionsvorrichtung zu steuern, um Photonen zu detektieren, die über mindestens einen der Photonen-Migrationspfade innerhalb des fetalen Gewebes gewandert bzw. migriert sind; und
einen Prozessor (PC), der angeschlossen ist, um Signale von dem Detektor zu empfangen, wobei der Prozessor zusammenwirkend mit der Steuervorrichtung angeordnet ist, um den Betrieb der Lichtquelle und des Lichtdetektors zu steuern, um Photonen zu detektieren, die über den Photonen-Migrationspfad gewandert sind, einschließlich dem fetalen Gewebe;

wobei der Prozessor auch angeordnet ist, um mindestens zwei Datensätze zu bilden, wobei ein erster der Datensätze das Blutvolumen in der fetalen Geweberegion repräsentiert und ein zweiter der Datensätze die Blutoxgenierung in der fetalen Geweberegion repräsentiert; wobei der Prozessor angeordnet ist, um die ersten und zweiten Datensätze zu korrelieren, um die untersuchte fetale Geweberegion zu charakterisieren.

**41.** Optisches System nach Anspruch 40, wobei die Steuervorrichtung und der Prozessor angeordnet sind, um die optischen Daten zu evaluieren und daraus folgend den Betrieb der Lichtquelle und des Lichtdetektors zu steuern, um zusätzliche optische Daten zu sammeln, die den Photonen entsprechen, die teilweise innerhalb des Hirngewebes des Feten gewandert sind.

**42.** Optisches System nach Anspruch 40 oder 41, wobei der Prozessor ferner angeordnet ist, um eine Pulsfrequenz des Feten zu bestimmen.

**43.** Optisches System nach Anspruch 40, 41 oder 42, wobei das optische Modul, welches die Lichtquelle und den Lichtdetektor aufweist, konstruiert ist, um in Kontakt mit dem Abdomen positioniert zu werden.

**44.** Optisches System nach Anspruch 43, welches ferner ein Barriere- bzw. Grenzmaterial aufweist, welches zwischen dem optischen Modul, welches die Lichtquelle und den Lichtdetektor aufweist, und der Haut gelegen ist.

**45.** Optisches System nach Anspruch 40, 41 oder 42, wobei das optische Modul eine Vielzahl von optischen Fasern bzw. Lichtwellenleitern aufweist, die eine optische Kopplung zwischen der Lichtquelle und dem Lichtdetektor und dem Abdomen aufweist.

**46.** Optisches System nach Anspruch 40, 41 oder 42, wobei das optische Modul konstruiert ist, um das Muster aufzuweisen, wobei jede Lichtquelle von mehreren Lichtdetektoren umgeben ist.

**47.** Optisches System nach Anspruch 46, wobei die Lichtdetektoren mindestens zwei symmetrische Pfade für jede Lichtquelle vorsehen.

**48.** Optisches System nach Anspruch 40, 41 oder 42, wobei das optische Modul konstruiert ist, um ein Muster aufzuweisen, wobei der Lichtdetektor von mehreren Lichtquellen umgeben ist.

**49.** Optisches System nach Anspruch 48, wobei die Lichtquellen mindestens zwei symmetrische Pfade für jeden Lichtdetektor vorsehen.

**50.** Optisches System nach Anspruch 40, 41, 42, 43, 45 oder 46, wobei der Prozessor ferner angeordnet ist, um die Hämoglobinoxygenierung des fetalen Gewebes zu bestimmen.

**51.** Optisches System nach Anspruch 40, 41, 42, 43, 45 oder 46, wobei das optische Modul zur Platzierung auf dem Abdomen konstruiert ist, und zwar auf der Grundlage der Ortung des Kopfes des Feten durch ein Ultraschallsystem, sodass die optischen Daten den Photonen entsprechen, die teilweise innerhalb des Hirngewebes des Feten gewandert sind.

**52.** Optisches System nach Anspruch 41, wobei der Prozessor angeordnet ist, um die Hämoglobinoxygenierung des Hirngewebes zu bestimmen.

**53.** Optische Vorrichtung nach Anspruch 41, wobei der Prozessor angeordnet ist, um eine Abbildung des Hirngewebes zu erzeugen.

**54.** Optisches System nach Anspruch 41, wobei der Prozessor angeordnet ist, um Abbildungen vom Blutvolumen in dem Hirngewebe und der Blutoxygenierung in dem Hirngewebe zu erzeugen.

**55.** Optisches System nach Anspruch 40, 41, 42, 43, 45 oder 46, wobei der Prozessor angeordnet ist, um die ersten und zweiten Datensätze durch Bestimmen der Kongruenz zwischen den Daten der zwei Sätze zu korrelieren.

**56.** Optisches System nach Anspruch 40, wobei der Prozessor programmiert ist, um die ersten und zweiten Datensätze als zweidimensionale Abbildungen anzuordnen und um die Kongruenz unter Verwendung der zweidimensionalen Abbildungen zu bestimmen.

**57.** Optisches System nach Anspruch 40, wobei der Prozessor programmiert ist, um die ersten und zweiten Datensätze als zweidimensionale Abbildungen anzuordnen, und um die Kongruenz unter Verwendung der folgenden Formel zu bestimmen:

$$I - \left( \frac{\text{maximale Überlappungsrestgröße}}{\text{maximales Signal des ausgewählten Gewebes}} \right) x\ 100$$

**58.** Optisches System nach Anspruch 40, 41 oder 42, wobei der Prozessor eingestellt ist, um aus dem Datensatz einen Bilddatensatz herzustellen durch Implementieren eines optischen Tomographie-Algorithmus.

**59.** Optisches System nach Anspruch 58, in dem der optische Tomographie-Algorithmus Faktoren einsetzt, die zu einer bestimmten Wahrscheinlichkeitsverteilung der Photonen, die dem Streuungscharakter des abzubildenden Gewebes zuordenbar ist, in Beziehung stehen.

**60.** Optisches System nach Anspruch 40, 41, 42, 43, 45 oder 46, welches ferner ein Bildschirmvorrichtung bzw. Anzeigevorrichtung aufweist, die konstruiert ist, um den Bilddatensatz von dem Prozessor zu empfangen und eine Abbildung anzuzeigen.

**61.** Optisches System nach Anspruch 40, 41, 42, 43, 45 oder 46, welches konstruiert ist, um Photonen mit zwei Wellenlängen einzuführen und zu detektieren, die ausgewählt sind, um ein Ansprechvermögen bzw. eine Empfindlichkeit auf einen Gewebebestandteil vorzusehen.

**62.** Optisches System nach Anspruch 40, 41, 42, 43, 45 oder 46, wobei die Lichtquelle Licht einer Wellenlänge emittiert, die auf ein endogenes Pigment empfindlich reagiert.

**63.** Optisches System nach Anspruch 62, wobei das endogene Pigment Hämoglobin ist.

**64.** Optisches System nach Anspruch 40, 41, 42, 43, 45 oder 46, wobei die Lichtquelle Licht einer Wellenlänge emittiert, die auf ein exogenes Pigment empfindlich reagiert.

**65.** Optisches System nach Anspruch 64, in dem das exogene Pigment ein ausgewähltes Kontrastmittel ist.

**66.** Optisches System nach Anspruch 40, 41 oder 42, welches ferner Folgendes aufweist:

einen ersten Oszillator, der konstruiert ist, um eine erste Trägerwellenform mit einer ersten Frequenz in der Größenordnung von $10^8$ Hz zu erzeugen, wobei die erste Frequenz eine Zeitcharakteristik besitzt, die mit der Zeitverzögerung der Photonenmigration zwischen den Eingangs- und Detektionsanschlüssen kompatibel ist; wobei die Lichtquelle an den ersten Oszillator gekoppelt ist und konstruiert ist, um Licht, welches durch die erste Wellenträgerform moduliert wird, zu erzeugen; einen Phasendetektor, der konstruiert ist, um die Veränderung der Wellenform des detektierten Lichtes relativ zu der Wellenform des eingeführten Lichtes zu bestimmen, und um daraus die Phasenverschiebung des detektierten Lichtes bei dieser Wellenlänge zu messen, wobei das phasenverschobene Licht eine Anzeige bildet für die Streuungs- oder Absorptionseigenschaften der untersuchten Gewaberegion; und

wobei der Prozessor angeordnet ist, um die Datensätze auf der Grundlage der gemessenen Phasenverschiebung zu bilden.

**67.** Optisches System nach Anspruch 66, welches ferner Folgendes aufweist:

einen zweiten Oszillator, der konstruiert ist, um eine zweite Wellenform mit einer zweiten Frequenz zu erzeugen;

wobei der Detektor angeordnet ist, um eine Referenzwellenform mit einer Referenzfrequenz zu empfangen, die um eine Frequenz in der Größenordnung von $10^3$ Hz zur ersten Frequenz versetzt ist, und um eine Signal, mit dieser Ausgleichsfrequenz bzw. Offset-Frequenz zu produzieren, die der detektierten Strahlung entspricht; und wobei der Phasendetektor angepasst ist, um bei dieser Offset-Frequenz das detektierte Licht mit dem eingeführten Licht zu vergleichen, und um daraus die Phasenverschiebung zu bestimmen.

**68.** Optisches System nach Anspruch 40, 41 oder 42, welches ferner Folgendes aufweist:

einen Oszillator, der konstruiert ist, um eine erste Trägerwellenform mit einer ausgewählten Frequenz zu erzeugen, die mit der Zeitverzögerung der Photonenmigration von dem Eingangsanschluss zu dem Detektionsanschluss kompatibel ist; wobei die Lichtquelle angeschlossen ist, um von dem Oszillator die Trägerwellenform zu empfangen, und konstruiert ist, um optisches Licht, welches auf diese Frequenz moduliert ist, zu erzeugen; einen Phasenteiler, der angeschlossen ist, um die Trägerwellenform von dem Oszillator zu empfangen und um erste und zweite Phasenreferenzsignale von vordefinierten im Wesentlichen unterschiedlichen Phasen zu erzeugen;

erste und zweite Doppelgegentaktmischer (double-balanced mixer), die angeschlossen sind, um von dem Phasenteiler die ersten bzw. zweiten Phasenreferenzsignale zu empfangen, und die angeschlossen sind, um von dem Detektor das Detektorsignal zu empfangen und um daraus ein In-Phase-Ausgangssignal bzw. ein Quadratur-Ausgangssignal zu erzeugen; und

wobei der Prozessor mit den Doppelgegentaktmischern verbunden ist und angeordnet ist, um das In-Phase-Ausgangssignal und das Quadratur-Ausgangssignal zu empfangen und um daraus die Datensätze zu bilden.

**69.** Optisches System nach Anspruch 68, worin der Prozessor angeordnet ist, um eine Phasenverschiebung ($\theta_\lambda$) zwischen dem Licht, das an dem Eingangsanschluss eingeführt wird, und dem Licht, das an dem Detektionsanschluss detektiert wird, vor dem Bilden des Datensatzes zu berechnen.

**70.** Optisches System nach Anspruch 68, worin der Prozessor angeordnet ist, um eine durchschnittliche Migrationspfadlänge der Photonen zu berechnen, die in dem untersuchten Gewebe zwischen dem optischen Eingangsanschluss und dem optischen Detektionsanschluss vor dem Bilden des Datensatzes gestreut sind.

**71.** Optisches System nach Anspruch 70, worin der Prozessor ferner die Pfadlänge beim Quantifizieren der Hämoglobinsättigung (Y) des untersuchten Gewebes einsetzt.

**72.** Optisches System nach Anspruch 68, worin der Prozessor angeordnet ist, um eine Signalamplitude ($A_\lambda$) zu berechnen, die als eine Quadratwurzel aus einer Summe der Quadrate des In-Phase-Ausgangssignals und des Quadratur-Ausgangssignals vor dem Bilden des Datensatzes bestimmt wird.

**73.** Optisches System nach Anspruch 72, welches ferner Folgendes aufweist:

einen Schmalband-Detektor, der angeschlossen ist, um von dem optischen Detektor das Detektorsignal zu empfangen und daraus ein Gleichstrom-Ausgangssignal zu erzeugen; und

wobei der Signalprozessor ferner einen Modulationsindex ($M_\lambda$) bestimmt als ein Verhältnis der Werte der Signalamplitude und der Signalamplitude plus dem Gleichstrom-Ausgangssignal.

**74.** Optisches System nach Anspruch 40, 41 oder 42, welches ferner Folgendes aufweist:

mindestens einen Oszillator, der konstruiert ist, um eine Trägerwellenform mit einer ausgewählten Frequenz zu erzeugen, wobei die Lichtquelle betriebsmäßig mit dem Oszillator verbunden ist, der konstruiert ist um Licht einer sichtbaren oder infraroten Wellenlänge zu erzeugen, wobei das Licht in der Intensität mit dieser Frequenz moduliert ist, um ein bekanntes Lichtmuster zu erhalten;

wobei die Steuervorrichtung konstruiert ist, um die emittierte Lichtintensität oder das Phasenverhältnis von Mustern zu steuern, die gleichzeitig von den multiplen Eingangsanschlüssen eingeführt werden, wobei die eingeführten Muster das resultierende Licht bilden, welches einen beträchtlichen Gradienten der Photonendichte in zumindest einer Richtung besitzt;
wobei das resultierende Licht über die Migrationspfade gestreut und absorbiert wird;
wobei der Detektor konstruiert und angeordnet ist, um über die Zeit hinweg das resultierende Licht zu detektieren, welches in dem Gewebe zu dem Detektionsanschluss gewandert ist; und
wobei der Prozessor ferner angeordnet ist, um die Signale von dem detektierten resultierenden Licht in Beziehung zum eingeführten Licht zu verarbeiten, um Datensätze zu erzeugen, die eine Anzeige bilden für den Einfluss des untersuchten Gewebes auf den beträchtlichen Gradienten der Photonendichte der resultierenden Strahlung.

**75.** Optisches System nach Anspruch 74, welches ferner einen Phasendetektor aufweist, der konstruiert ist, um die Phase des detektierten Lichtes zu detektieren, und um die Phase an den Prozessor zu liefern.

**76.** Optisches System nach Anspruch 74, welches ferner einen Amplitudendetektor aufweist, der konstruiert ist, um die Amplitude des detektierten Lichtes zu detektieren, und um die Amplitude an den Prozessor zu liefern.

**77.** Optisches System nach Anspruch 74, wobei das Phasenverhältnis von Lichtmustern, die von zwei Eingangsanschlüssen eingeführt werden, 180° beträgt.

**78.** Optisches System nach Anspruch 40, 41 oder 42, worin die Lichtquelle relativ lange Lichtimpulse erzeugt und der Prozessor Datensätze bildet durch Subtrahieren der Amplitude von den zwei Impulsen, die von den zwei Eingangsanschlüssen emittiert werden, die relativ zu dem einen Detektionsanschluss symmetrisch angeordnet sind.

**Revendications**

**1.** Procédé optique pour un examen transabdominal, in vivo, non invasif, de tissu de foetus comprenant :

prévoir un module optique (12, 14, 212) comprenant un réseau d'accès optiques d'entrée couplés optiquement à des sources de lumière ($S_1$, $S_2$, $S_3$,...) et des accès de détection couplés optiquement à des détecteurs de lumière ($D_1$, $D_2$, $D_3$, ....) ; disposés selon un motif géométrique choisi qui assure de multiples trajets de migration de photons à l'intérieur de l'utérus d'une femme enceinte sujet ;
placer le module optique à l'extérieur de l'abdomen de la femme enceinte sujet ;
introduire de la lumière visible ou infrarouge à partir d'au moins un des accès optiques d'entrée dans l'utérus et recevoir des photons qui ont migré dans l'utérus vers au moins un des accès de détection ;
détecter les photons reçus par au moins un détecteur optique couplé optiquement audit au moins un accès de détection ;
contrôler lesdites étapes d'introduction et de détection pour recueillir des données optiques correspondant à des photons de lumière qui ont migré partiellement à l'intérieur d'une région de tissu foetal ;
former au moins deux ensembles de données, un premier des ensembles de données représentant un volume sanguin dans la région de tissu foetal et un deuxième des ensembles de données représentant une oxygénation sanguine de la région de tissu foetal ; et
traiter lesdites données optiques en corrélant les premier et deuxième ensembles de données pour caractériser la région de tissu foetal.

**2.** Procédé optique selon la revendication 1, dans lequel le traitement comprend la détermination d'une oxygénation d'hémoglobine du tissu foetal.

**3.** Procédé optique selon la revendication 1, comprenant en outre un étalonnage du module optique.

**4.** Procédé optique selon la revendication 1, comprenant en outre une stimulation du tissu foetal.

**5.** Procédé optique selon la revendication 1, dans lequel le traitement comprend une surveillance d'une région de tissu sélectionnée du tissu foetal.

**6.** Procédé optique selon la revendication 5, dans lequel la surveillance comprend une surveillance pendant l'accouchement.

**7.** Procédé optique selon la revendication 1, dans lequel le traitement comprend une capture d'image d'une région de tissu sélectionnée du tissu foetal.

**8.** Procédé optique selon la revendication 1 ou 2, dans lequel le traitement comprend une corrélation des premier et deuxième ensembles de données en déterminant une congruence entre des données des deux ensembles.

**9.** Procédé optique selon la revendication 8, dans lequel le traitement comprend le fait d'ordonner les premier et deuxième ensembles de données sous forme d'images bidimensionnelles et la détermination de ladite congruence en utilisant les images bidimensionnelles.

**10.** Procédé optique selon la revendication 8, dans lequel le traitement comprend le fait d'ordonner les premier et deuxième ensembles de données sous forme d'images bidimensionnelles et la détermination de ladite congruence en utilisant la formule suivante :

$$ 1 - \left( \frac{résidu\ de\ recouvrement\ max\ imum}{signal\ de\ tissu\ sélectionné\ max\ imum} \right) x100 $$

**11.** Procédé optique selon la revendication 1, dans lequel le traitement comprend une détermination de la fréquence du pouls du foetus.

**12.** Procédé optique selon la revendication 1, dans lequel l'étape de contrôle comprend le recueil des données optiques correspondant à des photons qui ont migré partiellement à l'intérieur du tissu du cerveau du foetus.

**13.** Procédé optique selon la revendication 1, dans lequel l'étape de placement comprend le déplacement du module optique sur l'extérieur de l'abdomen pour réorienter les trajets de migration de photons à l'intérieur de l'utérus afin que les données optiques correspondent à des photons qui ont migré partiellement à l'intérieur du tissu du cerveau du foetus.

**14.** Procédé optique selon la revendication 1, comprenant en outre la localisation de la tête du foetus en utilisant un système à ultrasons et ensuite, en fonction de cette localisation, la réalisation du placement du module optique sur l'extérieur de l'abdomen pour positionner les trajets de migration de photons à l'intérieur de l'utérus afin que les données optiques correspondent à des photons qui ont migré partiellement à l'intérieur du tissu du cerveau du foetus.

**15.** Procédé optique selon la revendication 12, 13 ou 14, dans lequel le traitement comprend une détermination d'oxygénation d'hémoglobine du tissu du cerveau.

**16.** Procédé optique selon la revendication 12, 13, ou 14, dans lequel le traitement comprend une détermination de la fréquence du pouls du foetus.

**17.** Procédé optique selon la revendication 12, 13, ou 14, dans lequel le traitement comprend une évaluation du tissu du cerveau.

**18.** Procédé optique selon la revendication 12, 13 ou 14, dans lequel le traitement comprend la création d'une image du tissu du cerveau.

**19.** Procédé optique selon la revendication 18, dans lequel le traitement comprend la création d'images de volume sanguin dans le tissu du cerveau et d'oxygénation sanguine dans le tissu du cerveau.

**20.** Procédé optique selon la revendication 19, dans lequel les images comprennent des données d'oxygénation d'hémoglobine.

**21.** Procédé optique selon la revendication 19, dans lequel les images comprennent des données de désoxygénation d'hémoglobine.

**22.** Procédé optique selon la revendication 18, dans lequel les images comprennent des données sensibles à un constituant du tissu.

**23.** Procédé optique selon la revendication 12, 13 ou 14, dans lequel le traitement comprend la formation d'au moins deux ensembles de données, un premier des ensembles de données représentant un volume sanguin dans le tissu du cerveau et un deuxième des ensembles de données représentant une oxygénation sanguine du tissu du cerveau ; et le procédé comprenant en outre une corrélation des premier et deuxième ensembles de données pour détecter du tissu anormal dans le tissu du cerveau.

**24.** Procédé optique selon la revendication 1, 3, 4 ou 5, dans lequel la source de lumière émet de la lumière ayant une longueur d'onde sensible à un pigment endogène.

**25.** Procédé optique selon la revendication 24, dans lequel le pigment endogène est de l'hémoglobine.

**26.** Procédé optique selon la revendication 1, 3, 4 ou 5, dans lequel la source de lumière émet de la lumière ayant une longueur d'onde sensible à un pigment exogène.

**27.** Procédé optique selon la revendication 26, dans lequel le pigment exogène est un agent de contraste sélectionné.

**28.** Procédé optique selon la revendication 1 ou 2, comprenant en outre la génération d'une première forme d'onde de porteuse à une première fréquence de l'ordre de $10^8$ Hz, la première fréquence ayant une caractéristique temporelle compatible avec le retard temporel de la migration de photons entre les accès d'entrée et les accès de détection, le procédé comprenant en outre :

produire la lumière modulée par la première forme d'onde de porteuse ;
déterminer un changement dans la forme d'onde de la lumière détectée relatif à la forme d'onde de la lumière introduite et déterminer à partir de cela le déphasage de la lumière détectée à ladite longueur d'onde, la lumière déphasée étant indicative de propriétés de dispersion ou d'absorption de la région de tissu examinée ; et
former lesdits ensembles de données en fonction du déphasage mesuré.

**29.** Procédé optique selon la revendication 28, comprenant en outre la génération d'une deuxième forme d'onde à une deuxième fréquence, le procédé comprenant en outre :

fournir audit détecteur une forme d'onde de référence ayant un décalage fréquentiel de référence d'une fréquence de l'ordre de $10^3$ Hz par rapport à la première fréquence, produire un signal, à la fréquence décalée, correspondant à de la lumière des photons détectés ; et
comparer, à la fréquence décalée, la lumière détectée avec la lumière introduite et déterminer à partir de cela le déphasage.

**30.** Procédé optique selon la revendication 1 ou 2, comprenant en outre le fait de produire une première forme d'onde de porteuse ayant une fréquence sélectionnée compatible avec un retard temporel de migration de photons à partir de l'accès d'entrée jusqu'à l'accès de détection, prévoir un séparateur de phase connecté de façon à recevoir ladite forme d'onde de porteuse, et prévoir des premier et deuxième mélangeurs doublement équilibrés ; le procédé comprenant en outre :

fournir la forme d'onde de porteuse à l'une des sources de lumière et produire de la lumière modulée à ladite fréquence ;
produire par le séparateur de phase des premier et deuxième signaux de phase de référence ayant des phases sensiblement différentes prédéfinies ;
produire un signal de sortie en phase et un signal de sortie en quadrature par les premier et deuxième mélangeurs doublement équilibrés recevant les premier et deuxième signaux de phase de référence, respectivement, et
former lesdits ensembles de données en utilisant le signal de sortie en phase et le signal de sortie en quadrature.

**31.** Procédé optique selon la revendication 30, comprenant en outre le calcul d'un déphasage ($\theta_\lambda$) entre la lumière introduite et la lumière détectée pour former l'ensemble de données.

**32.** Procédé optique selon la revendication 30, comprenant en outre le calcul d'une longueur moyenne de trajet de migration de photons dispersés dans le tissu examiné entre les accès optiques d'entrée et de détection avant de former l'ensemble de données.

**33.** Procédé optique selon la revendication 32, comprenant en outre une quantification de saturation d'hémoglobine (Y) utilisant ladite longueur de trajet.

**34.** Procédé optique selon la revendication 30, comprenant en outre un calcul d'une amplitude de signal ($A_\lambda$) déterminée en tant que racine carrée d'une somme de carrés du signal de sortie en phase et du signal de sortie en quadrature avant de former l'ensemble de données.

**35.** Procédé optique selon la revendication 1 ou 2, comprenant en outre au moins un oscillateur conçu pour produire une forme d'onde de porteuse d'une fréquence sélectionnée, un contrôleur conçu pour commander l'intensité lumineuse émise ou la relation de phase de motifs introduits simultanément à partir de plusieurs accès d'entrée, lesdits motifs introduits formant de la lumière résultante qui possède un gradient notable de densité de photons dans au moins une direction, la lumière résultante étant dispersée et absorbée sur les trajets de migration ; le procédé comprenant en outre :

produire ladite lumière modulée en intensité à ladite fréquence pour obtenir un motif lumineux connu ;

détecter dans le temps la lumière résultante qui a migré dans le tissu ; et

traiter des signaux de ladite lumière résultante détectée en relation avec la lumière introduite pour créer les ensembles de données indicatifs de l'influence du tissu examiné sur le gradient notable de densité de photons de la lumière résultante.

36. Procédé optique selon la revendication 35, comprenant en outre la détection d'une phase de la lumière détectée.

37. Procédé optique selon la revendication 35, comprenant en outre la détection d'une amplitude de la lumière détectée.

38. Procédé optique selon la revendication 35, dans lequel la relation de phase entre des motifs lumineux introduits par deux accès d'entrée est de 180 degrés.

39. Procédé optique selon la revendication 1 ou 2, dans lequel les sources de lumière produisent des impulsions lumineuses relativement longues et le traitement comprend la soustraction de l'amplitude de deux desdites impulsions émises à partir de deux accès d'entrée situés symétriquement par rapport à un accès de détection donné.

40. Système optique (15, 45, 100, 150, 200, 260, 300) pour un examen transabdominal in vivo, non invasif, de tissu foetal, comprenant :

un module optique (12, 14, 212) comprenant un réseau d'accès optiques d'entrée et d'accès de détection disposés selon un motif géométrique sélectionné pour obtenir de multiples trajets de migration de photons à l'intérieur de l'utérus, chaque accès optique d'entrée étant conçu pour introduire de la lumière visible ou infrarouge émise par une source de lumière ($S_1$, $S_2$, $S_3$, ...), chaque accès de détection optique étant conçu pour recevoir des photons de lumière qui ont migré à partir d'au moins un des accès d'entrée et pour fournir la lumière reçue à un détecteur de lumière ($D_1$, $D_2$, $D_3$,...) ;

un contrôleur (19, 49, 140, 175, 262, 264), conçu et agencé pour commander le fonctionnement de la source lumineuse et du détecteur lumineux pour détecter des photons qui ont migré sur au moins un des trajets de migration de photons à l'intérieur du tissu foetal ; et

un processeur (PC) connecté de façon à recevoir des signaux dudit détecteur, le processeur étant agencé pour coopérer avec le contrôleur pour commander le fonctionnement de la source de lumière et du détecteur de lumière pour détecter des photons qui ont migré sur un trajet de migration de photons comprenant le tissu foetal, le processeur étant aussi agencé pour former au moins deux ensembles de données, un premier des ensembles de données représentant un volume sanguin dans la région de tissu foetal et un deuxième des ensembles de données représentant une oxygénation sanguine dans la région de tissu foetal; le processeur étant agencé pour corréler les premier et deuxième ensembles de données pour caractériser la région de tissu foetal examinée.

41. Système optique selon la revendication 40, dans lequel le contrôleur et le processeur sont agencés pour évaluer les données optiques et pour commander ensuite le fonctionnement de la source de lumière et du détecteur de lumière pour recueillir des données optiques supplémentaires correspondant à des photons qui ont migré partiellement à l'intérieur du tissu du cerveau du foetus.

42. Système optique selon la revendication 40 ou 41, dans lequel le processeur est en outre agencé pour déterminer la fréquence du pouls du foetus.

43. Système optique selon la revendication 40, 41 ou 42, dans lequel le module optique, comprenant la source de lumière et le détecteur de lumière, est conçu pour être positionné en contact avec l'abdomen.

44. Système optique selon la revendication 43, comprenant en outre un matériau de barrière placé entre le module optique, comprenant la source de lumière et le détecteur de lumière, et la peau.

45. Système optique selon la revendication 40, 41 ou 42, dans lequel le module optique comprend de multiples fibres optiques assurant un couplage optique entre la source de lumière et le détecteur de lumière et l'abdomen.

**46.** Système optique selon la revendication 40, 41 ou 42, dans lequel le module optique est conçu de façon à inclure ledit motif comportant chaque source de lumière entourée de plusieurs des détecteurs de lumière.

**47.** Système optique selon la revendication 46, dans lequel les détecteurs de lumière fournissent au moins deux trajets symétriques pour chaque source de lumière.

**48.** Système optique selon la revendication 40, 41 ou 42, dans lequel le module optique est conçu de façon à inclure le motif comportant chaque détecteur de lumière entouré de plusieurs des sources de lumière.

**49.** Système optique selon la revendication 48, dans lequel les sources de lumière fournissent au moins deux trajets symétriques pour chaque détecteur de lumière.

**50.** Système optique selon la revendication 40, 41, 42, 43, 45 ou 46, dans lequel le processeur est en outre agencé pour déterminer une oxygénation d'hémoglobine du tissu foetal.

**51.** Système optique selon la revendication 40, 41, 42, 43, 45 ou 46, dans lequel le module optique est conçu pour être placé sur l'abdomen sur la base de la localisation de la tête du foetus par un système à ultrasons afin que les données optiques correspondent à des photons qui ont migré partiellement à l'intérieur du tissu du cerveau du foetus.

**52.** Système optique selon la revendication 41, dans lequel le processeur est agencé pour déterminer une oxygénation d'hémoglobine du tissu du cerveau.

**53.** Dispositif optique selon la revendication 41, dans lequel le processeur est agencé pour créer une image du tissu du cerveau.

**54.** Système optique selon la revendication 41, dans lequel le processeur est agencé pour créer des images de volume sanguin dans le tissu du cerveau et d'oxygénation sanguine dans le tissu du cerveau.

**55.** Système optique selon la revendication 40, 41, 42, 43, 45 ou 46, dans lequel le processeur est agencé pour corréler les premier et deuxième ensembles de données en déterminant une congruence entre des données des deux ensembles.

**56.** Système optique selon la revendication 40, dans lequel le processeur est programmé pour ordonner les premier et deuxième ensembles de données sous forme d'images bidimensionnelles et pour déterminer ladite congruence en utilisant les images bidimensionnelles.

**57.** Système optique selon la revendication 40, dans lequel le processeur est programmé pour ordonner les premier et deuxième ensembles de données sous forme d'images bidimensionnelles et pour déterminer ladite congruence en utilisant la formule suivante :

$$1 - \left( \frac{\text{résidu de recouvrement maximum}}{\text{signal de tissu sélectionné maximum}} \right) x100$$

**58.** Système optique selon la revendication 40, 41 ou 42, dans lequel le processeur est apte à produire à partir de l'ensemble de données un ensemble de données d'images en mettant en oeuvre un algorithme de tomographie optique.

**59.** Système optique selon la revendication 58, dans lequel l'algorithme de tomographie optique utilise des facteurs associés à une distribution de probabilité déterminée de photons attribuable au caractère de dispersion du tissu en cours d'imagerie.

**60.** Système optique selon la revendication 40, 41, 42, 43, 45 ou 46, comprenant en outre un dispositif d'affichage conçu pour recevoir l'ensemble de données d'image à partir du processeur et pour afficher une image.

**61.** Système optique selon la revendication 40, 41, 42, 43, 45 ou 46, conçu pour introduire et détecter des photons à deux longueurs d'onde sélectionnées de façon à assurer une sensibilité à un constituant du tissu.

36

**62.** Système optique selon la revendication 40, 41, 42, 43, 45 ou 46, dans lequel la source de lumière émet de la lumière ayant une longueur d'onde sensible à un pigment endogène.

**63.** Système optique selon la revendication 62, dans lequel le pigment endogène est de l'hémoglobine.

**64.** Système optique selon la revendication 40, 41, 42, 43, 45 ou 46, dans lequel la source de lumière émet de la lumière ayant une longueur d'onde sensible à un pigment exogène.

**65.** Système optique selon la revendication 64, dans lequel le pigment exogène est un agent de contraste sélectionné.

**66.** Système optique selon la revendication 40, 41, ou 42 comprenant en outre :

un premier oscillateur conçu pour produire une première forme d'onde de porteuse à une première fréquence de l'ordre de $10^8$ Hz, la première fréquence ayant une caractéristique temporelle compatible avec le retard temporel de migration de photons entre les accès d'entrée et de détection ;
la source de lumière étant couplée au premier oscillateur et conçue pour produire de la lumière modulée par la première forme d'onde de porteuse ;
un détecteur de phase conçu pour déterminer un changement dans la forme d'onde de la lumière détectée relatif à la forme d'onde de la lumière introduite et pour mesurer à partir de cela le déphasage de la lumière détectée à ladite longueur d'onde, la lumière déphasée étant indicative de propriétés de dispersion ou d'absorption de la région de tissu examinée ; et
le processeur étant agencé pour former les ensembles de données sur la base du déphasage mesuré.

**67.** Système optique selon la revendication 66, comprenant en outre :

un deuxième oscillateur conçu pour produire une deuxième forme d'onde à une deuxième fréquence ;
le détecteur étant agencé pour recevoir une forme d'onde de référence ayant une fréquence de référence décalée d'une fréquence de l'ordre de $10^3$ Hz par rapport à la première fréquence et pour produire un signal, à la fréquence décalée, correspondant au rayonnement détecté ; et
le détecteur de phase étant apte à comparer, à la fréquence décalée, la lumière détectée et la lumière introduite et à déterminer à partir de cela le déphasage.

**68.** Système optique selon la revendication 40, 41 ou 42, comprenant en outre :

un oscillateur conçu pour produire une première forme d'onde de porteuse ayant une fréquence sélectionnée compatible avec un retard temporel de migration de photons à partir de l'accès d'entrée jusqu'à l'accès de détection ; la source de lumière étant connectée de façon à recevoir à partir de l'oscillateur ladite forme d'onde de porteuse et conçue pour produire de la lumière optique modulée à ladite fréquence ;
un séparateur de phase connecté de façon à recevoir la forme d'onde de porteuse provenant de l'oscillateur et à produire des premier et deuxième signaux de phase de référence ayant des phases sensiblement différentes prédéterminées ;
des premier et deuxième mélangeurs doublement équilibrés connectés de façon à recevoir à partir du séparateur de phase les premier et deuxième signaux de phase de référence, respectivement, et connectés de façon à recevoir à partir du détecteur le signal de détecteur et pour produire à partir de cela un signal de sortie en phase et un signal de sortie en quadrature, respectivement ; et
le processeur étant connecté aux mélangeurs doublement équilibrés et agencé pour recevoir le signal de sortie en phase et le signal de sortie en quadrature et former à partir de cela les ensembles de données.

**69.** Système optique selon la revendication 68, dans lequel le processeur est agencé pour calculer un déphasage ($\theta_\lambda$) entre la lumière introduite au niveau de l'accès d'entrée et la lumière détectée au niveau de l'accès de détection avant de former l'ensemble de données.

**70.** Système optique selon la revendication 68, dans lequel le processeur est agencé pour calculer une longueur moyenne de trajet de migration de photons diffusés dans le tissu examiné entre l'accès optique d'entrée et l'accès optique de détection avant de former l'ensemble de données.

**71.** Système optique selon la revendication 70, dans lequel le processeur utilise en outre la longueur de trajet dans la quantification de saturation d'hémoglobine (Y) du tissu examiné.

**72.** Système optique selon la revendication 68, dans lequel le processeur est agencé pour calculer une amplitude de signal ($A_\lambda$) déterminée en tant que racine carrée d'une somme de carrés du signal de sortie en phase et du signal de sortie en quadrature avant de former l'ensemble de données.

**73.** Système optique selon la revendication 72, comprenant en outre :

un détecteur à bande étroite connecté de façon à recevoir à partir du détecteur optique le signal de détecteur et à produire à partir de cela un signal de sortie continu (DC) ; et
le processeur de signal déterminant en outre un indice de modulation ($M_\lambda$) en tant que rapport entre des valeurs de l'amplitude du signal et de l'amplitude du signal plus le signal de sortie continu (DC).

**74.** Système optique selon la revendication 40, 41 ou 42, comprenant en outre :

au moins un oscillateur conçu pour produire une forme d'onde de porteuse ayant une fréquence sélectionnée, la source de lumière étant fonctionnellement connectée à l'oscillateur conçu pour produire de la lumière ayant une longueur d'onde visible ou infrarouge, la lumière étant modulée en intensité à ladite fréquence pour obtenir un motif lumineux connu ;
le contrôleur étant conçu pour commander l'intensité lumineuse émise ou la relation de phase de motifs introduits simultanément à partir de plusieurs accès d'entrée, les motifs introduits formant de la lumière résultante qui possède un gradient notable de densité de photons dans au moins une direction, la lumière résultante étant dispersée et absorbée sur les trajets de migration ;
le détecteur étant conçu et agencé pour détecter dans le temps la lumière résultante qui a migré dans le tissu vers l'accès de détection, et
le processeur étant en outre agencé pour traiter des signaux de la lumière résultante détectée en relation avec la lumière introduite pour créer les ensembles de données indicatifs de l'influence du tissu examiné sur le gradient notable de densité de photons du rayonnement résultant.

**75.** Système optique selon la revendication 74, comprenant en outre un détecteur de phase conçu pour détecter la phase de la lumière détectée et pour fournir la phase au processeur.

**76.** Système optique selon la revendication 74, comprenant en outre un détecteur d'amplitude conçu pour détecter l'amplitude de la lumière détectée et pour fournir ladite amplitude au processeur.

**77.** Système optique selon la revendication 74, dans lequel la relation de phase entre des motifs lumineux introduits par deux accès d'entrée est de 180 degrés.

**78.** Système optique selon la revendication 40, 41 ou 42, dans lequel la source de lumière produit des impulsions lumineuses relativement longues et le processeur forme les ensembles de données en soustrayant l'amplitude de deux des impulsions émises à partir de deux accès d'entrée situés symétriquement par rapport à un accès de détection.

FIG. 1A

FIG. 1

■ — DETECTOR
● — SOURCE: 750nm, 830nm

FIG. 1B

TOTAL: 9 DUAL λ SOURCES
4 PMT DETECTORS

FIG. 1C

FIG. 2A

FIG. 2

■ — DETECTOR
● — SOURCE: 750nm, 830nm

FIG. 2B

## 50 MHZ SINGLE WAVELENGTH PMS/PHASE ARRAY IMAGE SYSTEM

FIG. 3

EP 1 054 620 B1

FIG. 3A

| SOURCES | DETECTORS |
|---|---|
| 1, 2, 1a AND 2a | 1 |
| 2, 3, 2a, AND 3a | 2 |
| 4, 5, 4a, AND 5a | 1 |
| 5, 6, 5a, AND 4a | 2 |
| 7, 8, 7a, AND 8a | 3 |
| 8, 9, 8a, AND 9a | 4 |
| 4, 5, 4a, AND 5a | 3 |
| 5, 6, 5a, AND 6a | 4 |
| 7, 8, 7a, AND 8a | 3 |

40

# FIG. 3B

FIG. 4

EP 1 054 620 B1

HOMODYNE PHASE CANCELLATION IMAGER
(ONE 2S ID PART OF IMAGER)

FIG. 4A

FIG. 5

FIG. 6

SIGNAL AND DARK CURRENT

DIFFERENTIAL DETECTION WITH SIGNAL AND NOISE SUBSTRACTION

LIGHT INTENSITY CONTROL

LAMP FREQUENCY

OUTPUT

RESET

HOLDING

EP 1 054 620 B1

FIG. 6A

AIR COOLED LAMP

230

FAN

236
232A
232B
234

FIG. 7

AIR COOLED LIGHT GUIDE

FIBER
COUPLING

242

240
248

AIR
INLET

FIG. 7A

INFLOWING
JETS
244
246

AIR IN
252B

AIR COOLED BARRIER

LIGHT SOURCE
254

AIR IN
252A

250

FIG. 7B

HOLLOW
BARRIER
252

FIG. 8

EP 1 054 620 B1

## CIRCUIT CONFIGURATION FOR
## TIME MULTIPLEX APLITUDE CANCELLATION IMAGER

ONE ELEMENT
OF AMPLITUDE
CANCELLATION IMAGER

$S_1, S_2$     754nm
$S_{1A}, S_{2A}$   830nm
$G_{1-4}$    500μSEC GATE

## FIG. 8A

272

TIME SEQUENCE $S_1, S_1$

◄— 500μSEC —►◄— 500μSEC —►

$S_1$      $S_1$      $S_1$

50μSEC      50μSEC      50μSEC

$S_2$      $S_2$

$D_3$   $D_3$      $D_3$   $D_3$

$D_{11}$   $D_{11}$      $D_{11}$   $D_{11}$

$G_1$

GATE

DIFFERENCE $[D_3$-$D_{11}]S_1$

$S_2$      $S_2$

50μSEC      50μSEC

FIG. 8B

TIME MULTIPLEX

4 X 2 SOURCES, 21 DETECTORS, SH, AGC

FIG. 8C

FREQUENCY MULTIPLEX
21 X 2 SOURCES/OSCILLATORS, 4 DETECTORS, 40 FILTERS (HIGH/LOW), 20 AGC, 20 LOCK-IN

FIG. 8D

DUAL WAVELENGTH NIR PHASED ARRAY IMAGER
2D VASCULOGENESIS VS HYPERMETABOLISM IMAGERY

FIG. 9

## REFERENCES CITED IN THE DESCRIPTION

### Patent documents cited in the description

- WO 9313395 A
- US 5564417 A
- US 5551424 A
- US 5807263 A
- US 5673701 A
- US 5853370 A
- US 5119815 A
- US 5386827 A
- US 9600235 W
- US 9611630 W
- US 4972331 A
- US 5402778 A
- US 9305868 W
- WO 932514 A
- US 5553614 A
- US 5187672 A
- US 9204153 W
- WO 9220273 A
- US 5218962 A
- US 4869254 A
- US 4846183 A
- US 4700708 A
- US 4576173 A

### Non-patent literature cited in the description

- **Folkman J.** Tumor Angiogenesis: Therapeutic Implications. *N. Engl. Jour. Med.,* 1971, vol. 285, 1182-1186
- **Peebles, D.M. et al.** Changes in human fetal cerebral hemoglobin concentration and oxygenation during labor measured by near-infrared spectroscopy. *Am. J. Obstet. Gynecol,* 1992, vol. 166, 1369-73
- **Aldrich, C.J. et al.** Late fetal heart rate decelerations and changes in cerebral oxygenation during the first stage of labour. *Br. J. Obstet. Gynaecol.,* 1995, vol. 102, 9-13
- **Aldrich, C.J. et al.** Fetal heart rate changes and cerebral oxygenation measured by near infrared spectroscopy during the first stage of labour. *E. J. Obstet. Gynecol. Reprod. Biol.,* 1996, vol. 64, 189-195
- **Peebles, D.M. et al.** Relation between frequency of uterine contractions and human fetal cerebral oxygen saturation studied during labour by near-infrared spectroscopy. *Br. J. Obstet. Gynaecol.,* 1994, vol. 101, 44-48
- **Aldrich, C.J. et al.** Fetal cerebral oxygenation measured by near-infrared spectroscopy shortly before birth and acid-base status at birth. *Obstet. Gynecol.,* 1994, vol. 84, 861-6
- **Aldrich, C.J. et al.** The effect of maternal oxygen administration on human fetal cerebral oxygenation measured during labour by near infrared spectroscopy. *Br. J. Obstet. Gynaecol,* 1994, vol. 101, 509-513
- **Aldrich, C.J. et al.** The effect of maternal posture on fetal cerebral oxygenation measured during labour by near infrared spectroscopy. *Br. J. Obstet. Gynaecol.,* 1995, vol. 102, 14-19
- **Mannheimer, P.D. et al.** Physio-optical considerations in the design of fetal pulse oximetry sensors. *Euro. J. Obstet. & Gyn.,* 1997, S9-S 19